# EUROPÄISCHE PATENTSCHRIFT

(11) **EP 0 453 837 B1**
(45) Veröffentlichungstag und Bekanntmachung des Hinweises auf die Patenterteilung: **19.06.1996**
(21) Anmeldenummer: 91105395.7
(22) Anmeldetag: 05.04.1991
(51) Int. Cl.: C07D 405/12, C07D 311/58, A01N 43/58

(54) **Substituierte Pyridazinone, Verfahren zu ihrer Herstellung und ihre Verwendung als Schädlingsbekämpfungsmittel**
Substituted pyridazinones, process for their preparation and their use as parasiticide
Pyridazinones substitués, procédé pour leur préparation et leur utilisation comme parasiticide

(30) Priorität: 18.04.1990 DE 4012338
(43) Veröffentlichungstag der Anmeldung: 30.10.1991
(73) Patentinhaber: BAYER AG, 51368 Leverkusen (DE)
(72) Erfinder: Weissmüller, Joachim, Dr., W-4019 Monheim (DE); Erdelen, Christoph, Dr., W-5653 Leichlingen 3 (DE); Wachendorff-Neumann, Ulrike Dr., W-4019 Monheim (DE); Stendel, Wilhelm, Dr., W-5600 Wuppertal 1 (DE); Leicht, Wolfgang, Dr., W-5090 Leverkusen 1 (DE)

(56) Entgegenhaltungen:
- EP-A- 0 193 853
- EP-A- 0 373 425
- CHEMICAL ABSTRACTS, vol. 110, no. 26, 1989, Columbus, Ohio, US; abstract no. 57425M, '2h-benzopyran derivatives' Seite 665 ; Spalte 2 ; & CS-A-252 601

## Beschreibung

Die Erfindung betrifft neue substituierte Pyridazinone, mehrere Verfahren zu ihrer Herstellung und ihre Verwendung als Schädlingsbekämpfungsmittel.

Es ist bekannt, daß bestimmte substituierte Pyridazinone, wie beispielsweise die Verbindung 2-t-Butyl-4-chlor-5-{2-[4-(3,3-dimethylbutyl)-2,6-dichlorphenoxy]-ethylthio}-pyridazin-(2H)-3-on, die Verbindung 2-t-Butyl-4-chlor-5-(4-t-butyl-phenylmethylthio)-3(2H)-pyridazinon oder die Verbindung 2-t-Butyl-4-chlor-5-[2-(4-methyl-2,6-dichlorphenoxy)-ethylthio]-pyridazin-(2H)-3-on gute Wirksamkeit gegen Schädlinge, insbesondere eine gute insektizide, akarizide, nematizide und fungizide Wirksamkeit besitzen (vgl. z.B. EP 232 825 und EP 134 439). Die Wirkungshöhe bzw. Wirkungsdauer dieser vorbekannten Verbindungen ist jedoch, insbesondere gegen bestimmte Organismen oder bei niedrigen Anwendungskonzentrationen nicht in allen Anwendungsgebieten völlig zufriedenstellend.

Es wurden neue substituierte Pyridazinone der allgemeinen Formel (Ia), 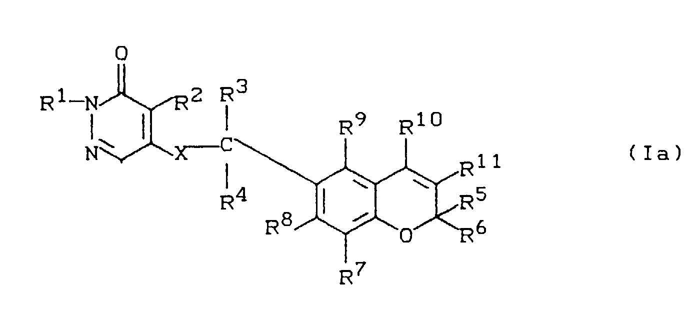 in welcher
- R¹: für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxyalkyl, (Di)alkylaminoalkyl, Alkylthioalkyl mit 1 bis 6 Kohlenstoffatomen in den jeweiligen Alkylteilen, für geradkettiges oder verzweigtes Alkenyl mit 2 bis 8 Kohlenstoffatomen, für geradkettiges oder verzweigtes 1 bis 6 Halogenatome enthaltendes Halogenalkenyl mit 2 bis 8 Kohlenstoffatomen, für jeweils gegebenenfalls einfach bis vierfach, gleich oder verschieden substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten Alkylteil steht, wobei als Substituenten im Cycloalkylteil jeweils infrage kommen: Alkyl mit 1 bis 4 Kohlenstoffatomen und/ oder Halogen; R¹ ferner für gegebenenfalls einfach bis vierfach, gleich oder verschieden substituiertes Aryl oder Aralkyl mit 6 bis 10 Kohlenstoffatomen in den jeweiligen Arylteilen und gegebenenfalls 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
- R²: für Fluor, Chlor, Brom, Iod oder für geradkettiges oder verzweigtes Alkyl mit 1 bis 4 Kohlenstoffatomen steht,
- R³ und R⁴: unabhängig voneinander jeweils für Wasserstoff oder für geradkettiges oder verzweigtes Alkyl mit 1 bis 4 Kohlenstoffatomen stehen,
- R⁵ und R⁶: unabhängig voneinander jeweils für Wasserstoff, jeweils geradkettiges oder verzweigtes und gegebenenfalls durch Alkoxy, Carboxy, Alkoxycarbonyl, oder (Di)alkylamino substituiertes Alkyl mit jeweils 1 bis 10 Kohlenstoffatomen in den Alkylteilen, für geradkettiges oder verzweigtes Alkenyl mit 2 bis 10 Kohlenstoffatomen, für Carboxy oder für Alkoxycarbonyl mit 1 bis 4 Kohlenstoffatomen im Alkylteil, für jeweils gegebenenfalls ein- bis dreifach, gleich oder verschieden substituiertes Phenyl oder Phenylalkyl mit 1 bis 3 Kohlenstoffatomen im Alkylteil, wobei als Substituenten vorzugsweise Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio infrage kommen, stehen, oder
- R⁵ und R⁶: gemeinsam mit dem Kohlenstoffatom, an das sie gebunden sind, einen gegebenenfalls substituierten drei- bis siebengliedrigen gesättigten oder ungesättigten Carbocyclus bilden,
- R⁷, R⁸, R⁹, R¹⁰ und R¹¹: unabhängig voneinander jeweils für Wasserstoff, Halogen, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, oder Halogenalkylthio mit jeweils 1 bis 4 Kohlenstoffatomen und 1 bis 9 gleichen oder verschiedenen Halogenatomen stehen und
- X: für Sauerstoff oder Schwefel steht,
gefunden.

Weiterhin wurde gefunden, daß man die neuen substituierten Pyridazinone der allgemeinen Formel (Ia), erhält, wenn man
(a) 5-Hydroxy- oder 5-Mercaptopyridazinone der Formel (II), 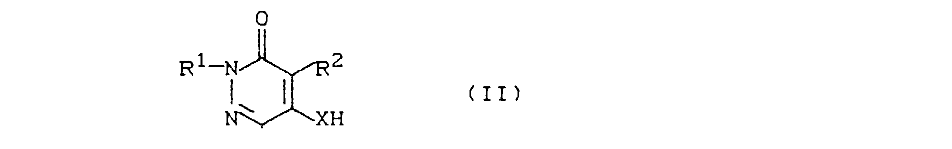 in welcher
   X, R¹ und R² die oben angegebene Bedeutung haben,
   mit Alkylierungsmitteln der Formel (III), 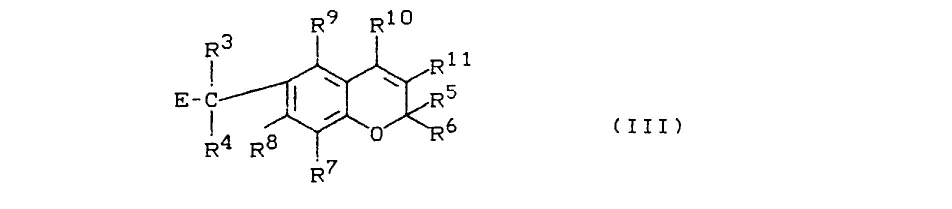 in welcher
   - E: für eine elektronenanziehende Abgangsgruppe steht und
   - R³, R⁴, R⁵, R⁶, R⁷, R⁸, R⁹, R¹⁰ und R¹¹: die oben angegebene Bedeutung haben,
   gegebenenfalls in Gegenwart eines Verdünnungsmittels und gegebenenfalls in Gegenwart eines Reaktionshilfsmittels umsetzt oder wenn man
(b) 5-Halogenpyridazinone der Formel (IV), 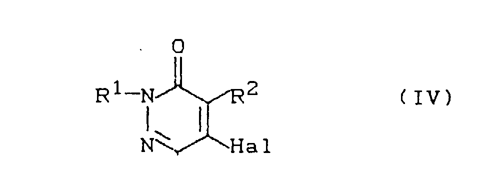 in welcher
   R¹ und R² die oben angegebene Bedeutung haben und
   Hal für Halogen steht,
   mit Alkoholen oder Thiolen der Formel (V), 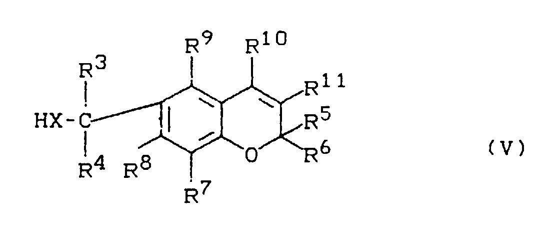 in welcher
   X, R³, R⁴, R⁵, R⁶, R⁷, R⁸, R⁹, R¹⁰ und R¹¹ die oben angegebene Bedeutung haben,
   gegebenenfalls in Gegenwart eines Verdünnungsmittels und gegebenenfalls in Gegenwart eines Reaktionshilfsmittels umsetzt.

Schließlich wurde gefunden, daß die neuen substituierten Pyridazinone der allgemeinen Formel (Ia) gute Wirksamkeit gegen Schädlinge, insbesondere gute insektizide, akarizide und ovizide Wirksamkeit besitzen.

Überraschenderweise zeigen die erfindungsgemäßen substituierten Pyridazinone eine erheblich bessere insektizide Wirksamkeit gegen pflanzenschädigende und warmblüter-parasitierende Insekten und Spinnentiere sowie zusätzlich eine bessere ovizide Wirkung, als die aus dem Stand der Technik bekannten substituierten Pyridazinone, wie beispielsweise die Verbindung 2-t-Butyl-4-chlor-5-{2-[4-(3,3-dimethylbutyl)-2,6-dichlorphenoxy]-ethylthio}-pyridazin-(2H)-3-on, die Verbindung 2-t-Butyl-4-chlor-5-(4-t-butyl-phenylmethylthio)-3(2H)-pyridazinon oder die Verbindung 2-t-Butyl-4-chlor-5-[2-(4-methyl-2,6-dichlorphenoxy)-ethylthio]-pyridazin-3(2H)-on, welche chemisch und wirkungsmäßig naheliegende Verbindungen sind.

Die erfindungsgemäßen substituierten Pyridazinone sind durch die Formel (Ia) allgemein definiert. Bevorzugt sind Verbindungen der Formel (Ia), bei welchen
- R¹: für Methyl, Ethyl, n- oder i-Propyl, n-, i-, s - oder t-Butyl, Methoxymethyl, Ethoxymethyl, Methoxyethyl oder Ethoxyethyl, Methylthiomethyl, Ethylthiomethyl, Methylthioethyl, Ethylthioethyl, Methylaminomethyl, Methylaminoethyl, Dimethylaminomethyl, Dimethylaminoethyl, Ethylaminomethyl, Ethylaminoethyl, Diethylaminomethyl, Diethylaminoethyl, 1 bis 3 bzw. 1 bis 5 Fluor- und/oder Chloratome enthaltendes Halogenmethyl, Halogenethyl, n- oder i-Halogenpropyl, n- oder i-Halogenbutyl, Allyl, n- oder i-Butenyl, n- oder i-Pentenyl, 2-Fluorpropen-3-yl, 2-Chlorpropen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl oder für gegebenenfalls ein- bis vierfach gleich oder verschieden im Cycloalkylteil substituiertes Cyclopentyl, Cyclohexyl, Cyclopropylmethyl, Cyclopentylmethyl, Cyclopentylethyl, Cyclohexylmethyl oder Cyclohexylethyl steht, wobei als Substituenten jeweils infrage kommen: Fluor, Chlor, Brom, Methyl, Ethyl, Methoxy, Ethoxy, Halogenmethyl, Halogenethyl, Halogenmethoxy oder Halogenethoxy, wobei Halogen bevorzugt für Fluor und/oder Chlor steht; weiterhin für gegebenenfalls ein- oder zweifach gleich oder verschieden im Phenylteil substituiertes Phenyl, Benzyl oder Phenethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Trifuormethyl, Difluormethyl sowie Trifluormethoxy,
- R²: für Chlor, Brom, Methyl, Ethyl, n- oder i-Propyl steht,
- R³ und R⁴: unabhängig voneinander jeweils für Wasserstoff, Methyl oder Ethyl stehen,
- R⁵ und R⁶: unabhängig voneinander jeweils für Wasserstoff, Methyl, Ethyl, n- oder i-Propyl, n-, i- oder t-Butyl, n- oder i-Pentyl, n- oder i-Hexyl, Dimethoxymethyl, Carboxy, Methoxycarbonyl, Ethoxycarbonyl, n- oder i-Propoxycarbonyl, n-, i- oder t-Butoxycarbonyl, 2-Carboxy-ethyl, 2-Methoxycarbonylethyl, 2-Ethoxycarbonyl-ethyl, 2-t-Butoxycarbonylethyl, 4-Carboxy-butyl, 4-Methoxycarbonyl-butyl, 3-Diethylamino-propyl, Allyl, 1,1-Dimethylpropen-3-yl, 1,1-Dimethylbuten-4-yl, 2-Pyrrolidinyl-ethyl, jeweils gegebenenfalls ein- bis dreifach gleich oder verschieden substituiertes Phenyl oder Phenylalkyl mit 1 bis 3 Kohlenstoffatomen im Alkylteil, wobei als Phenylsubstituenten jeweils infrage kommen: Methyl, Ethyl, n- oder i-Propyl, n-, i-oder t-Butyl, Fluor, Chlor, Brom, Methoxy, Ethoxy, Trifluormethyl, Trifluormethoxy, Difluormethoxy, Trifluormethylthio, oder
- R⁵ und R⁶: gemeinsam mit dem Kohlenstoffatom, an das sie gebunden sind, einen jeweils gegebenenfalls einfach bis dreifach gleich oder verschieden substituierten Cyclopropyl-, Cyclopentyl-, Cyclohexyl-, Cyclohexenyl- oder Cycloheptylring bilden, wobei als Substituenten infrage kommen: Methyl, Ethyl, n- oder i-Propyl, n-, i- oder t-Butyl und
- R⁷, R⁸, R⁹, R¹⁰ und R¹¹: unabhängig voneinander jeweils für Wasserstoff, Fluor, Chlor, Brom, Methyl, Ethyl, Methoxy, Ethoxy, Methylthio, Ethylthio, Trifluormethyl, Trifluormethoxy, Trifluormethylthio oder Difluormethoxy stehen und
- X: für Sauerstoff oder Schwefel steht.

Im einzelnen seien außer den bei den Herstellungsbeispielen genannten Verbindungen die folgenden substituierten Pyridazinone der allgemeinen Formel (Ia) genannt:

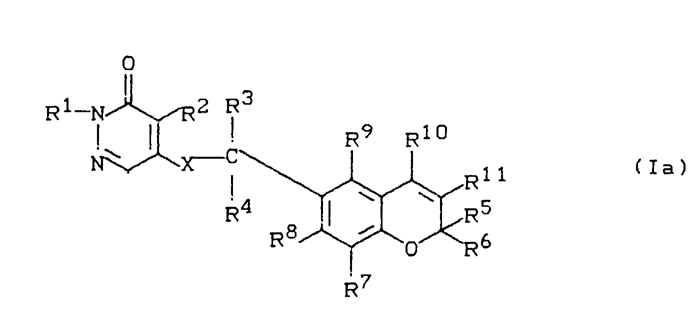

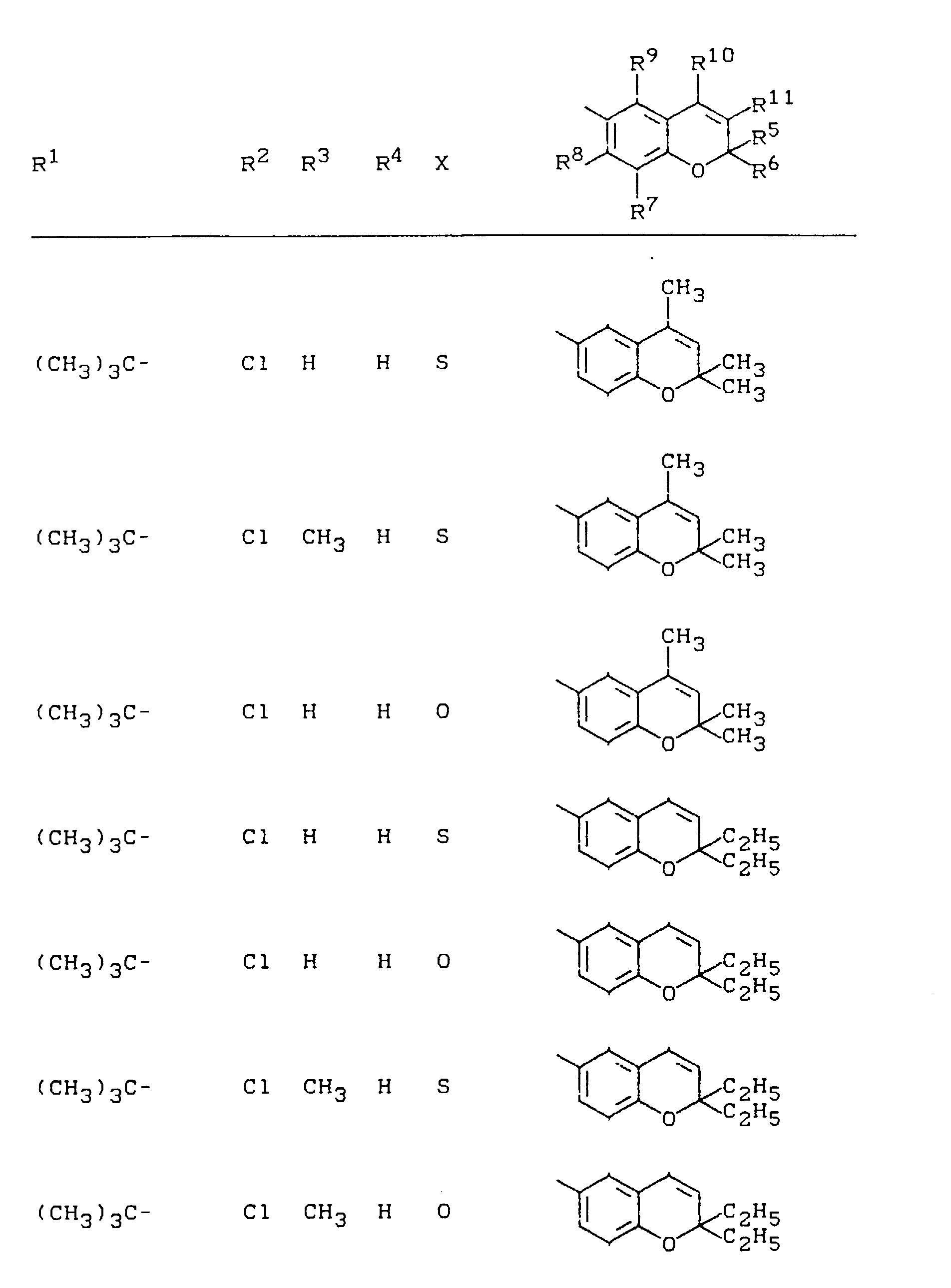

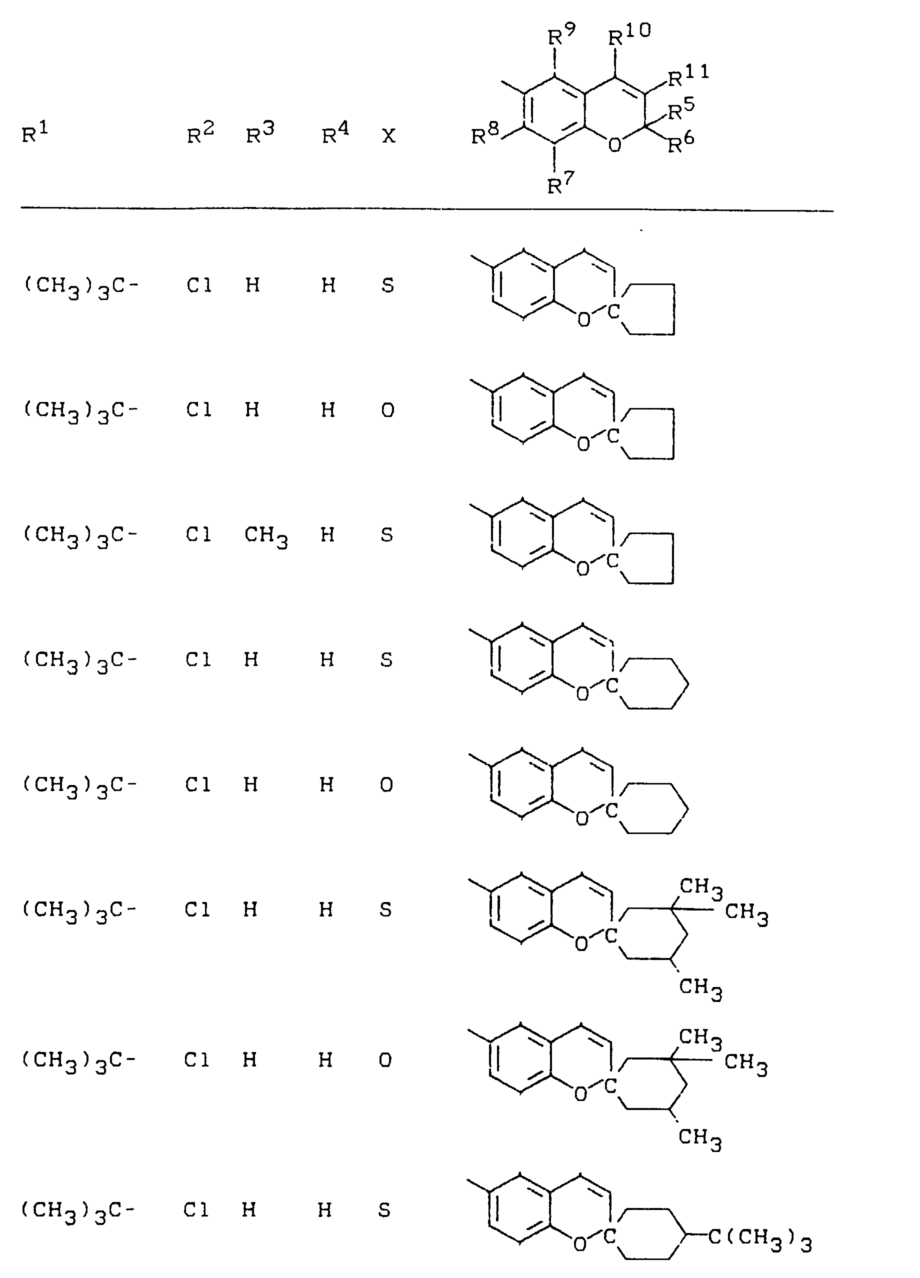

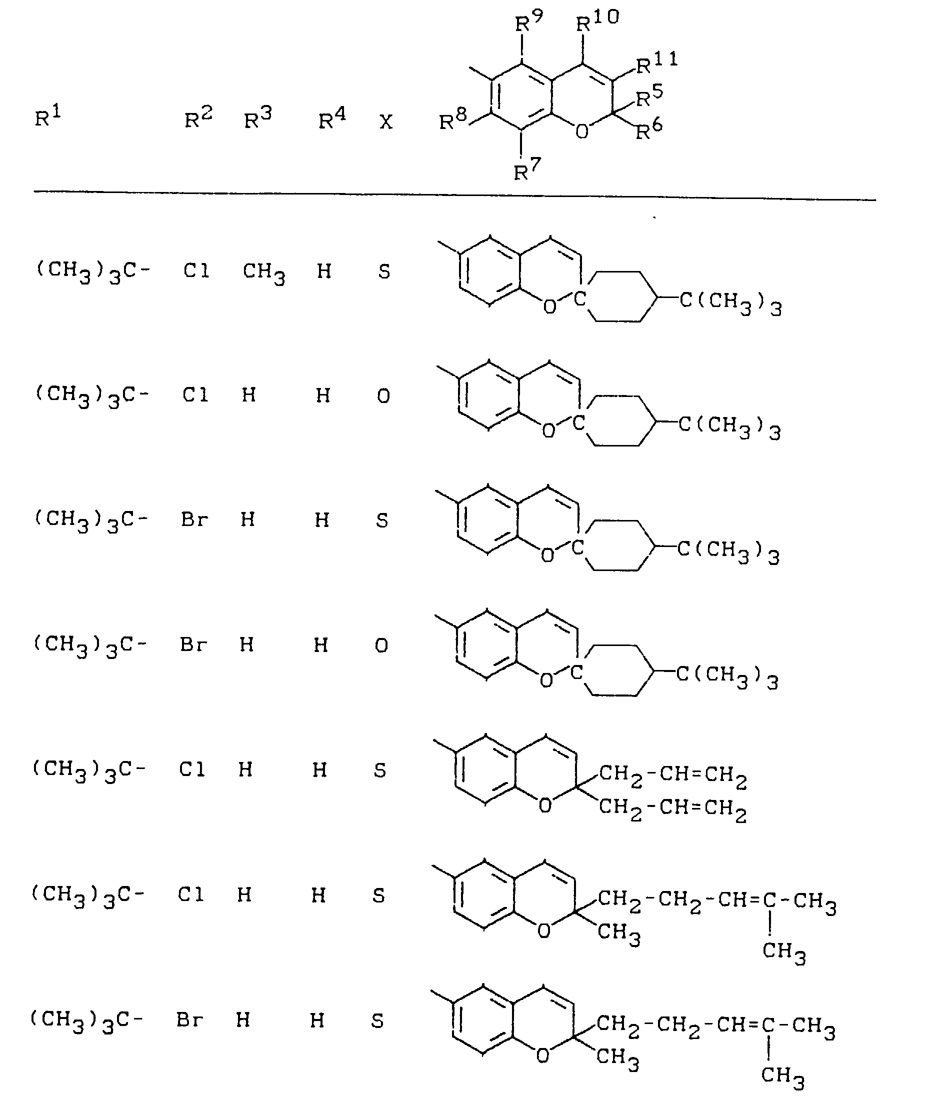

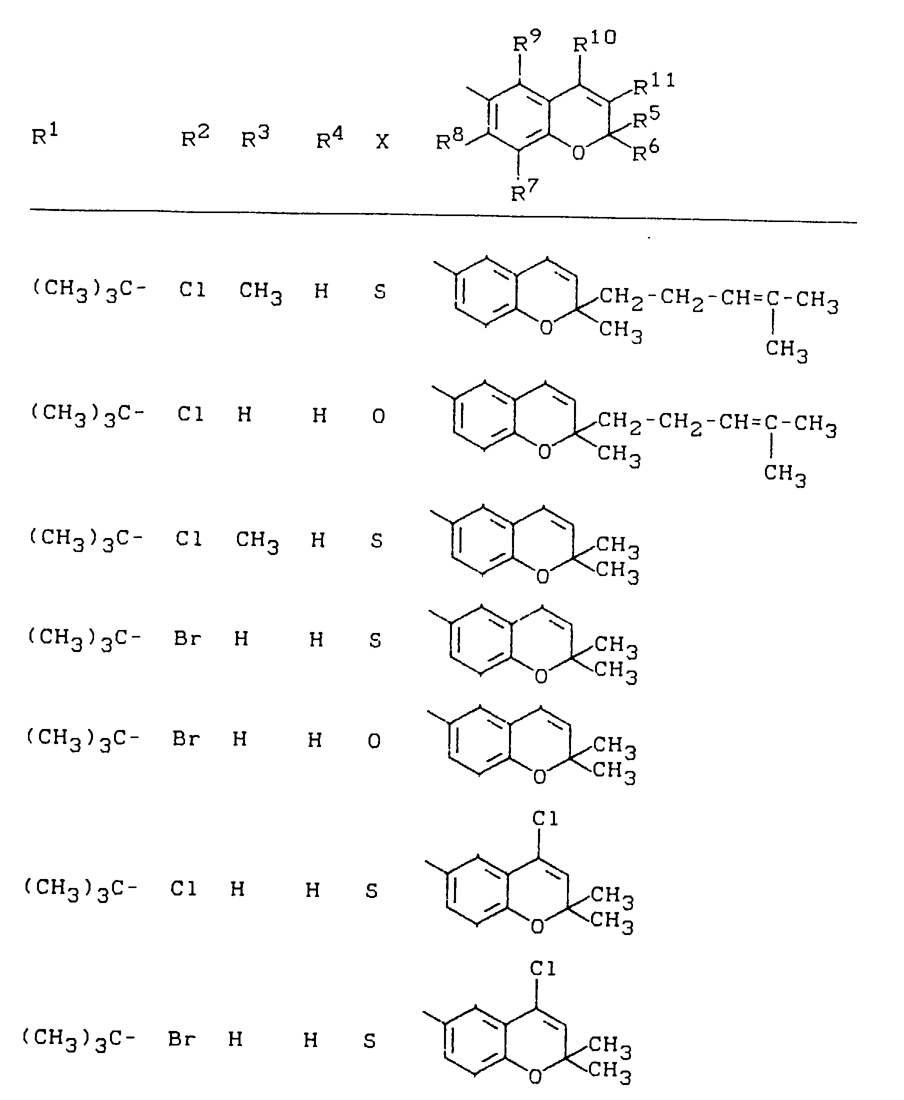

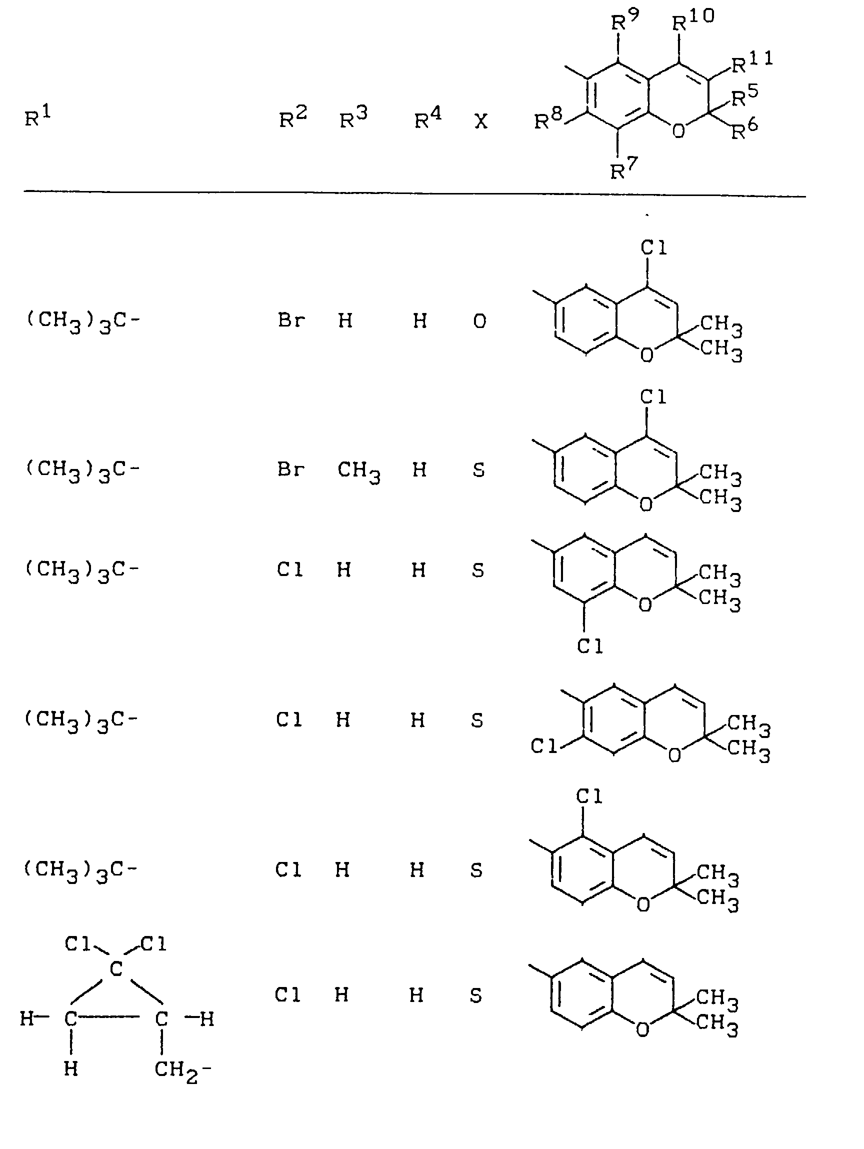

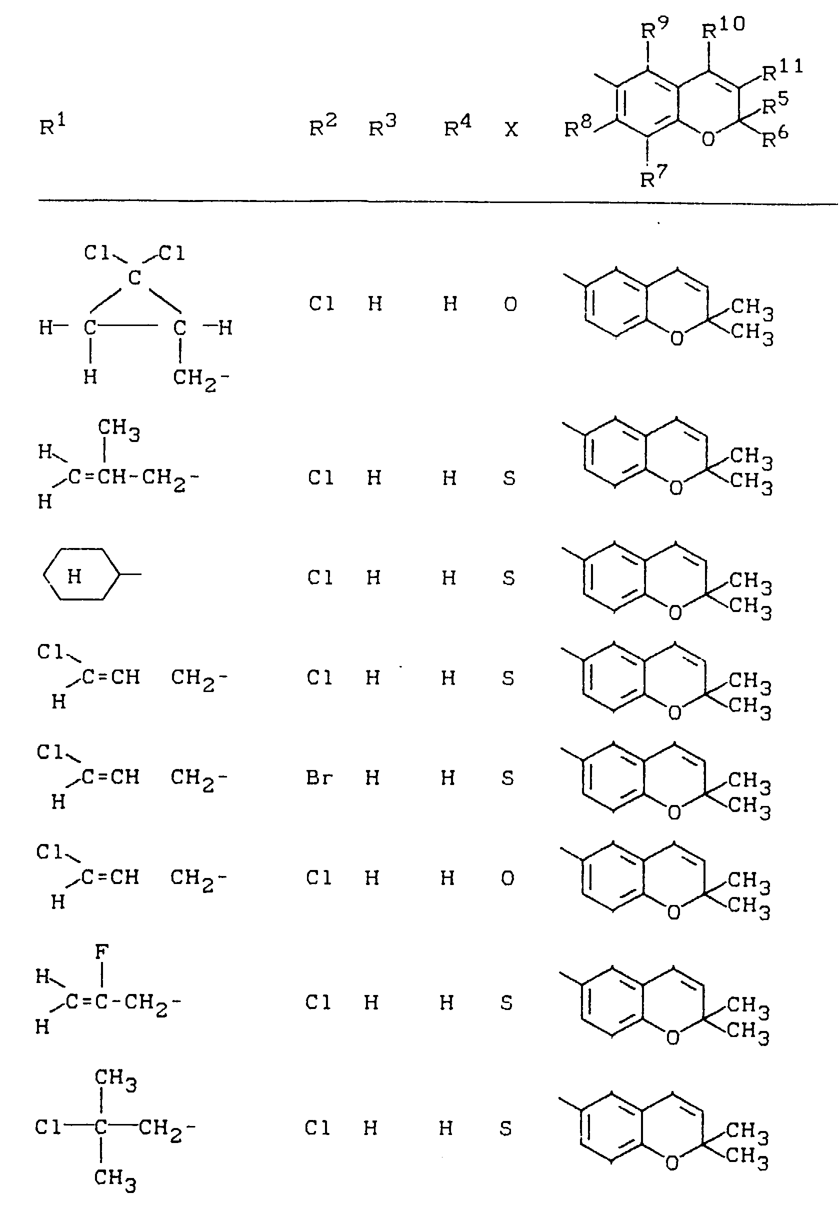

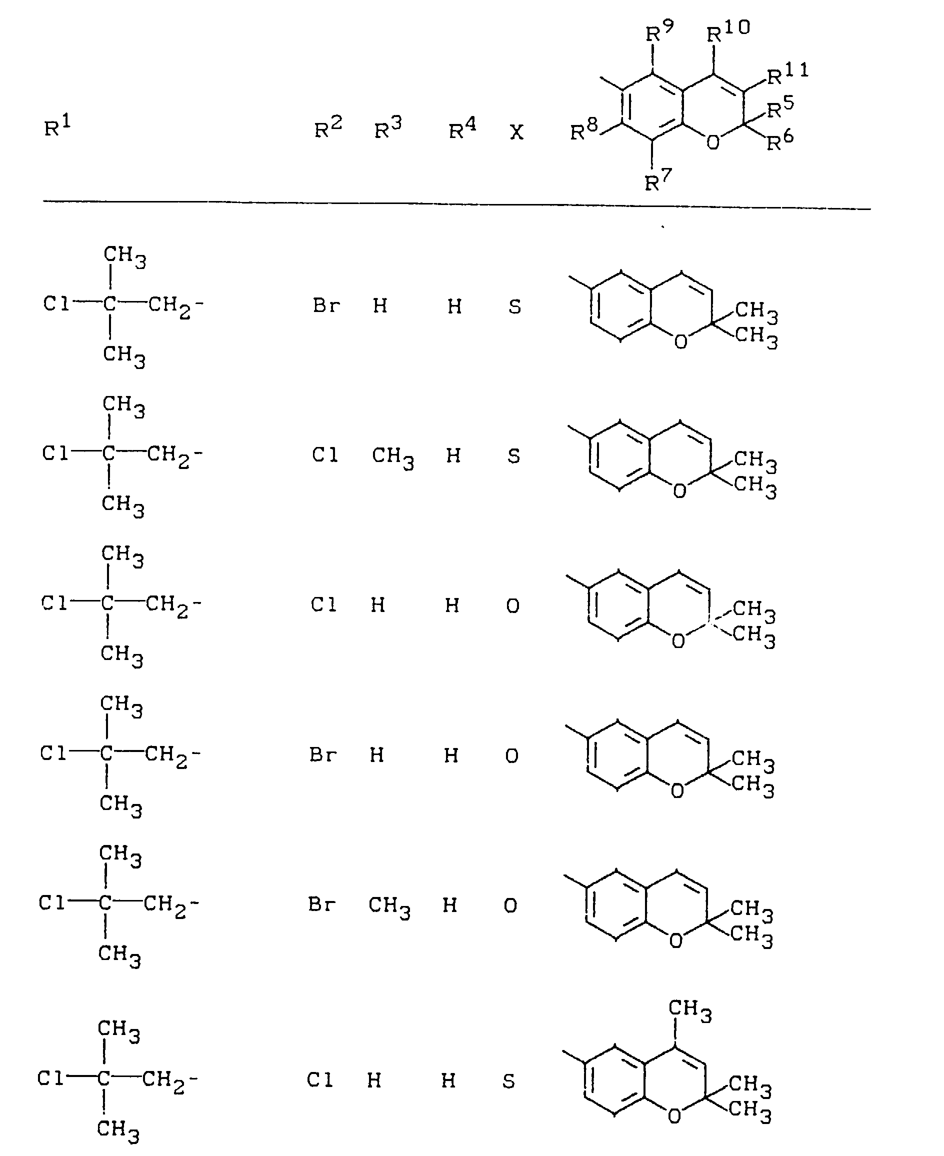

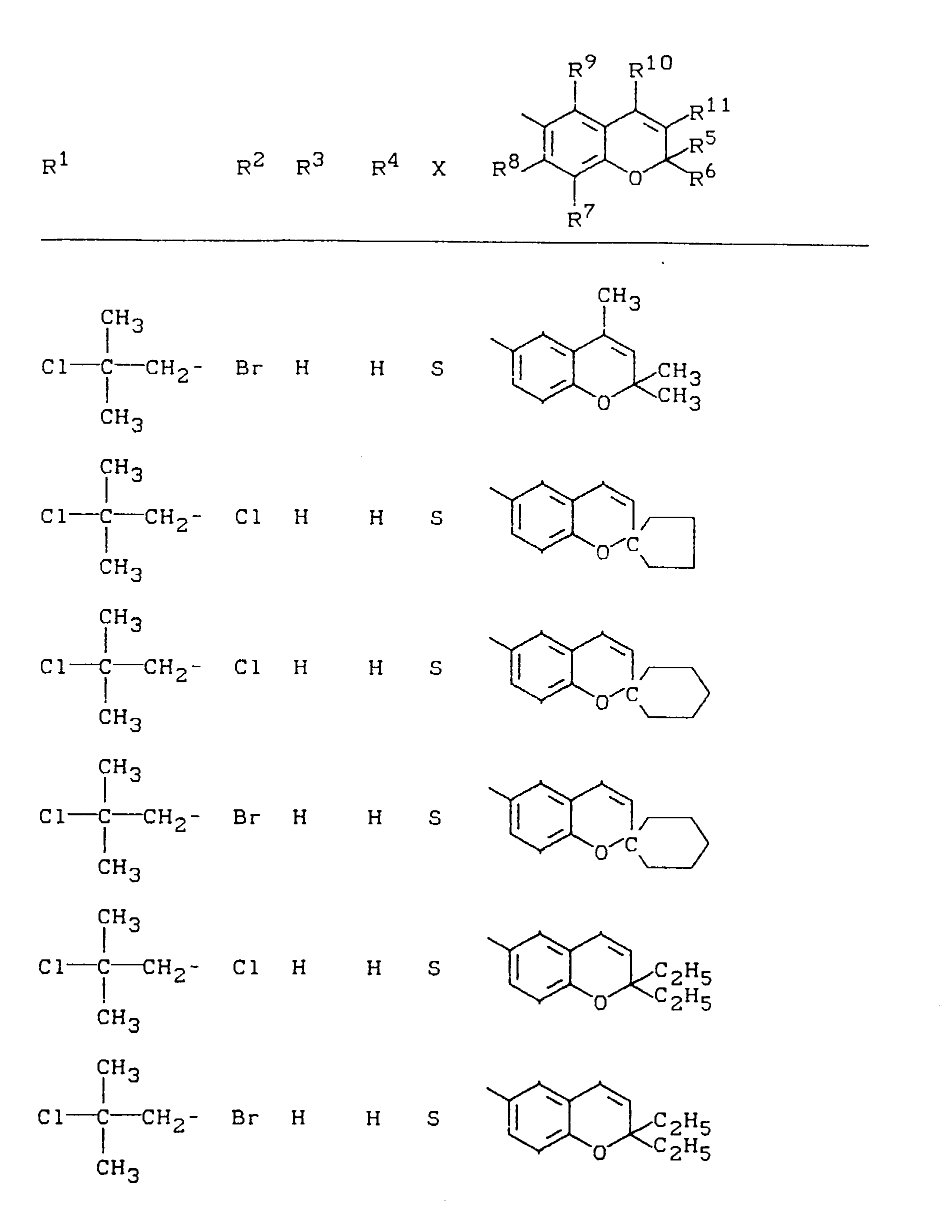

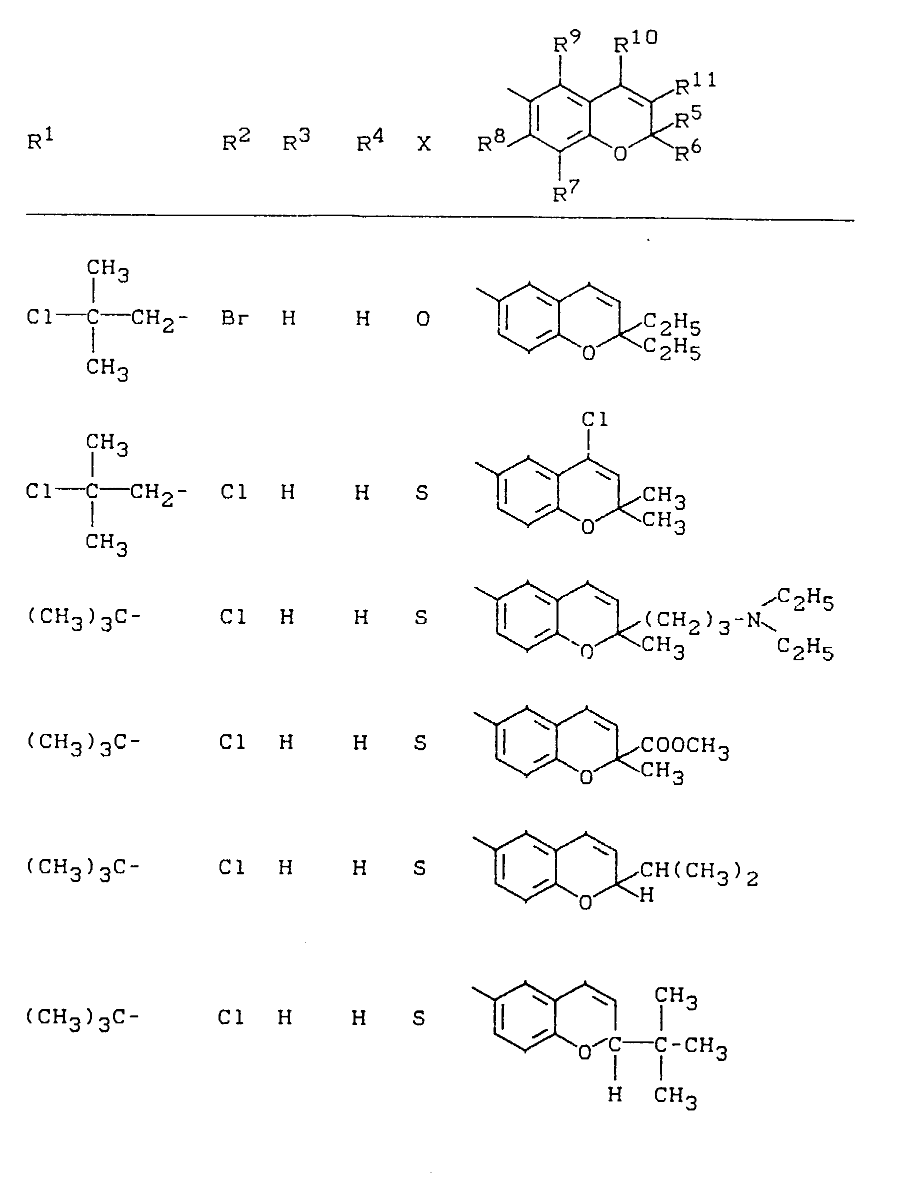

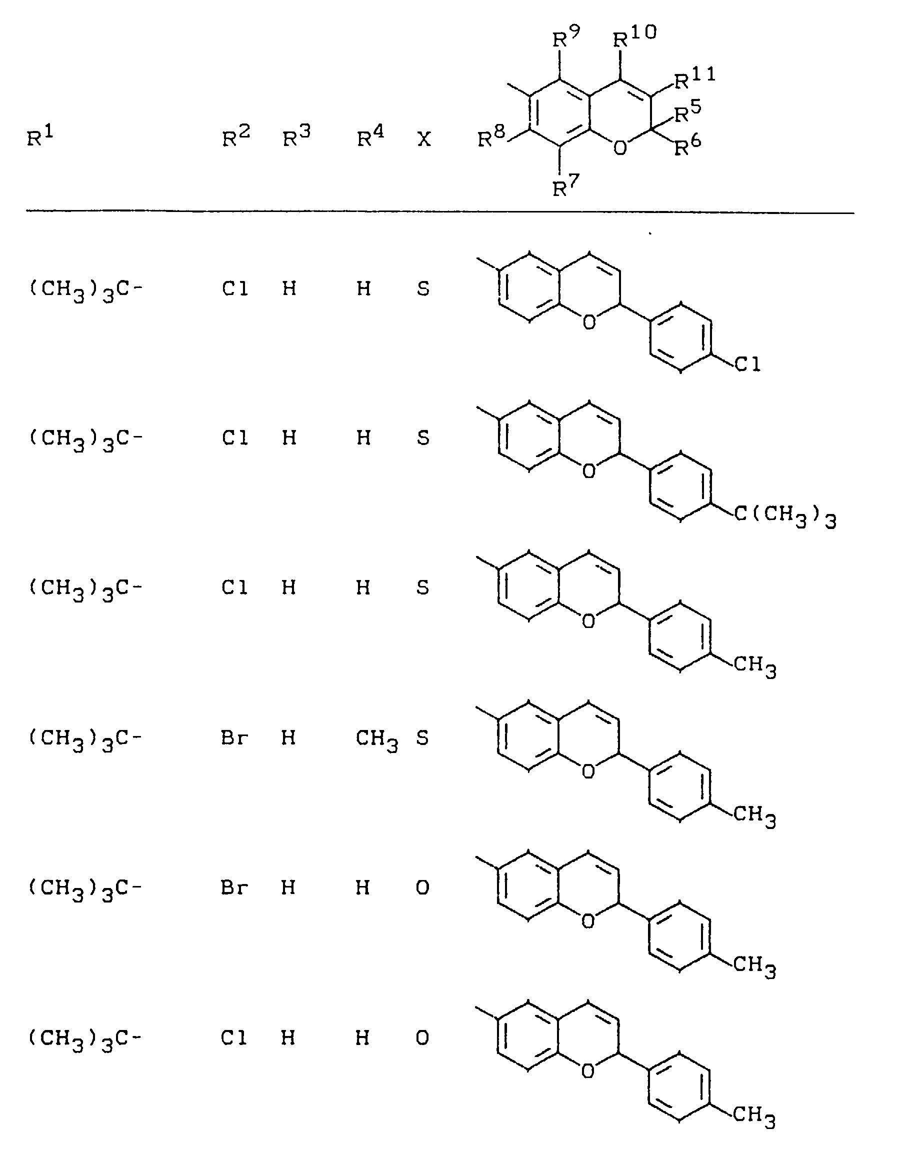

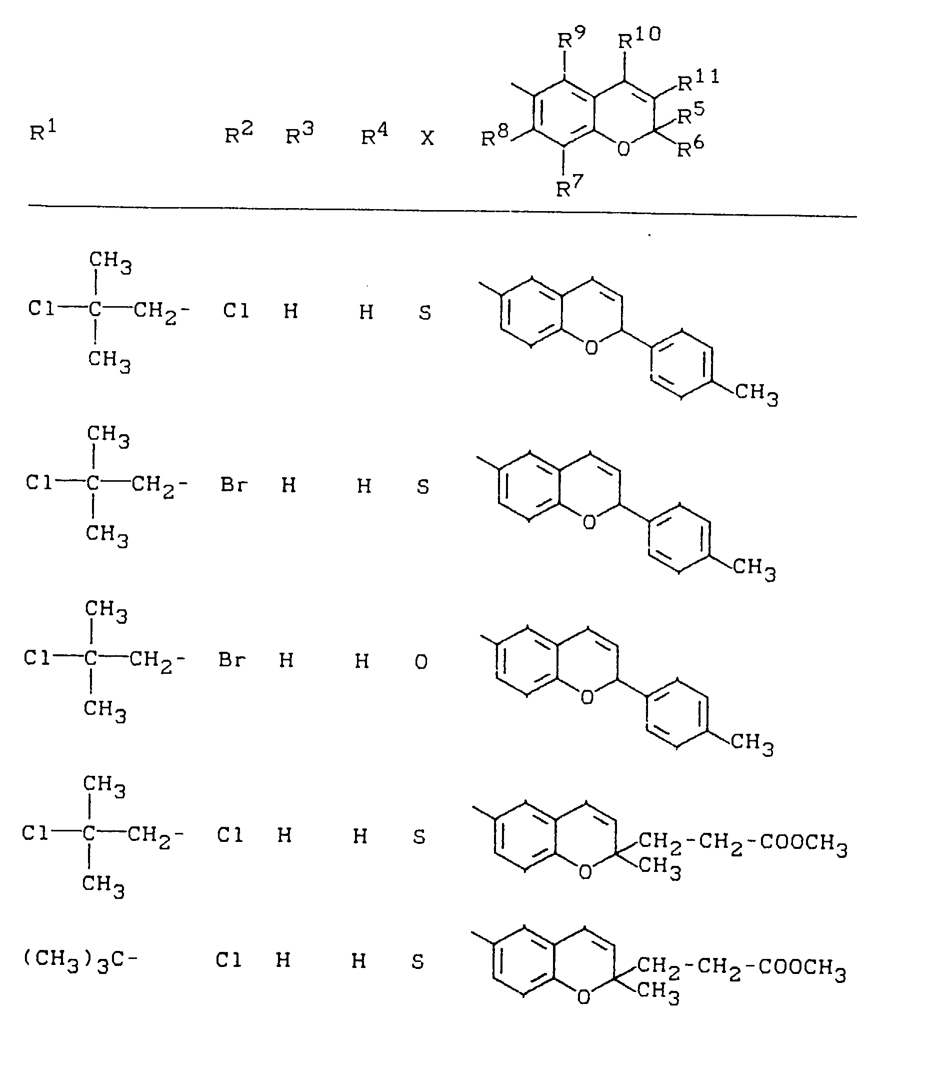

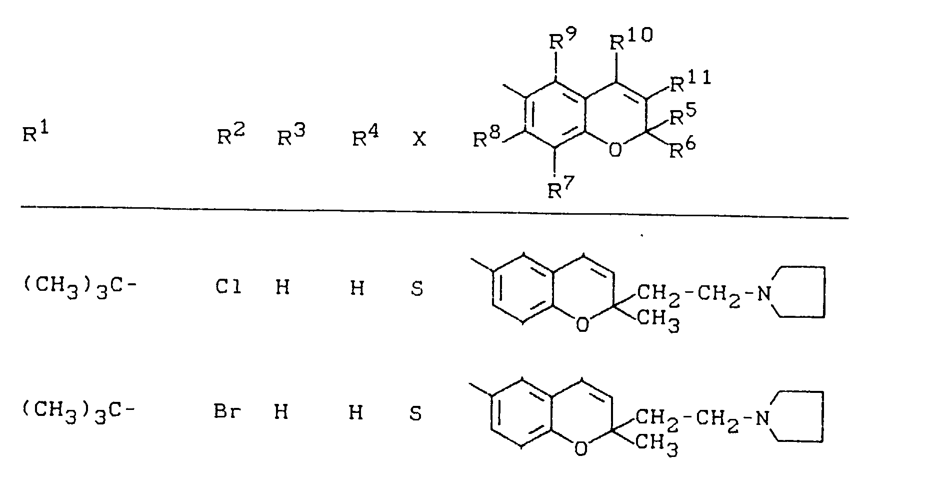

Verwendet man beispielsweise 2-t-Butyl-4-chlor-5-hydroxy-pyridazin-3-(2H)-on und 6-Chlormethyl-2,2-dimethyl-(2H)-chromen als Ausgangsstoffe, so läßt sich der Reaktionsablauf des erfindungsgemäßen Verfahrens (a) durch das folgende Formelschema darstellen: 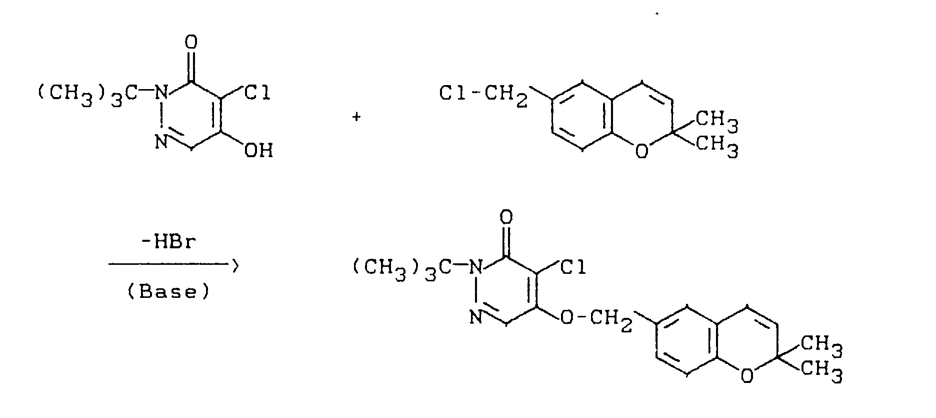 Verwendet man beispielsweise 2-Cyclopropylmethyl-4,5-dichlorpyridazin-3-(2H)-on und 6-Mercaptomethyl-2,2-dimethyl-(2H)-chromen als Ausgangsstoffe, so läßt sich der Reaktionsablauf des erfindungsgemäßen Verfahrens (b) durch das folgende Formelschema darstellen: 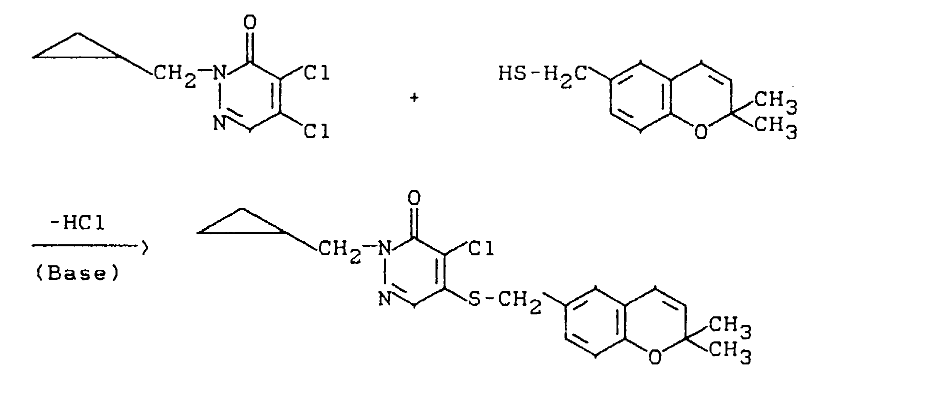 Die zur Durchführung des erfindungsgemäßen Verfahrens (a) als Ausgangsstoffe benötigten 5-Hydroxy- oder 5-Mercaptopyridazinone sind durch die Formel (II) allgemein definiert. In dieser Formel (II) stehen R¹ R² und X vorzugsweise für diejenigen Reste, die bereits im Zusammenhang mit der Beschreibung der erfindungsgemäßen Stoffe der Formel (Ia) als bevorzugt für diese Substituenten genannt wurden.

Die 5-Hydroxy- und 5-Mercaptopyridazinone der Formel (II) sind bekannt oder erhältlich in Analogie zu bekannten Verfahren (vgl. z.B. EP 302 346; EP 183 212; Chem. Pharm. Bull. 18, 147-156 [1979]; JP 61/109777; Heterocycles 26, 1 - 4 [1987]; Pestic. Sci. 9, 571 - 581 [1978]; Chem. Zvesti 30, 663 - 673 [1976] bzw. CA 87: 135236y; CS 146172 vom 15.12.1972).

Die zur Durchführung des erfindungsgemäßen Verfahrens (a) weiterhin als Ausgangsstoffe benötigten Alkylierungsmittel sind durch die Formel (III) allgemein definiert. In dieser Formel (III) stehen R³, R⁴, R⁵, R⁶, R⁷, R⁸, R⁹, R¹⁰ und R¹¹ vorzugsweise für diejenigen Reste, die bereits im Zusammenhang mit der Beschreibung der erfindungsgemäßen Stoffe der Formel (Ia) als bevorzugt für diese Substituenten genannt wurden.

E steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxy-sulfonyloxy oder p-Toluolsulfonyloxy.

Die Alkylierungsmittel der Formel (III) sind allgemein bekannte Verbindungen oder erhältlich in Analogie zu allgemein bekannten Verfahren der organischen Chemie (vgl. DE-OS 2450193, CS-P 252601 - zit. in Chem. Abstr. 110, 57425).

Noch nicht bekannt sind die Alkylierungsmittel der Formel (III) 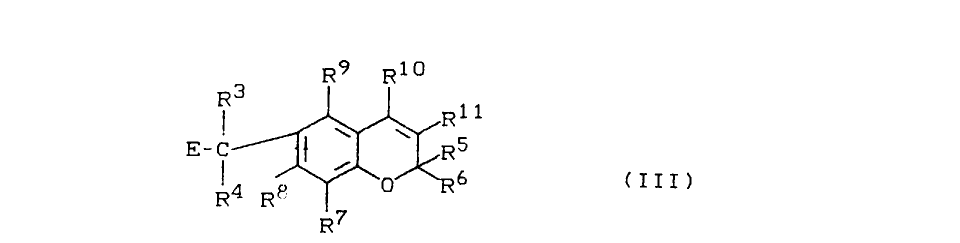 in welcher
- R³, R⁴, R⁵, R⁶, R⁷, R⁸, R⁹, R¹⁰ und R¹¹: die oben angegebene Bedeutung haben und
- E: für Halogen, wie vorzugsweise Chlor, Brom oder Iod sowie für jeweils gegebenenfalls substituiertes Alkylsulfonyloxy, Alkoxysulfonyloxy oder Arylsulfonyloxy wie vorzugsweise Methansulfonyloxy, Trifluormethansulfonyloxy, Methoxysulfonyloxy, Ethoxysulfonyloxy oder p-Toluolsulfonyloxy steht, mit der Ausnahme von 6-Chlormethyl-2H-1-benzopyran und 6-(1-Bromopropyl)-2,2-dimethyl-2H-1-benzopyran.

Die Alkylierungsmittel der Formel (III) werden in Analogie zu allgemein bekannten Verfahren erhalten, indem man entsprechende Alkohole der Formel (Va) 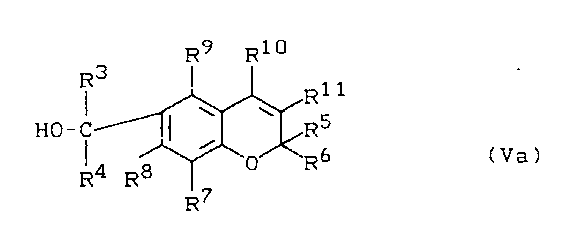 in welcher
- R³, R⁴, R⁵, R⁶, R⁷, R⁸, R⁹, R¹⁰ und R¹¹: die oben angegebene Bedeutung haben, mit entsprechenden Halogenierungs- bzw. Sulfonylierungsmitteln umsetzt (vgl. auch die Herstellungsbeispiele).

Die Alkohole der Formel (Va) sind erhältlich in Analogie zu allgemein bekannten Verfahren der organischen Chemie (vgl. Indian J. Chem. 23B, 1124 (1984); Liebigs Ann. Chem. 1986, 799; Indian J. Chem. 17B, 73 (1979); Houben-Weyl IX, 7-19; J. Nat. Prod. 49 (1), 143).

Die Alkohole der Formel (Va) 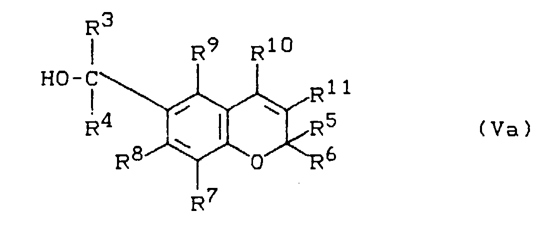 in welcher
- R³, R⁴, R⁵, R⁶, R⁷, R⁸, R⁹, R¹⁰ und R¹¹: die oben angegebene Bedeutung haben, mit der Maßgabe, daß R⁷, R⁸ oder R⁹ nicht für Methoxy stehen oder R⁷, R⁸, R⁹, R¹⁰ und R¹¹ nicht für Wasserstoff stehen, wenn R⁵ und R⁶ für Methyl stehen, sind neu.

Die Alkohole der Formel (Va) werden in Analogie zu allgemein bekannten Verfahren erhalten, indem man entsprechend substituierte Carbonylverbindungen der Formel (VI) 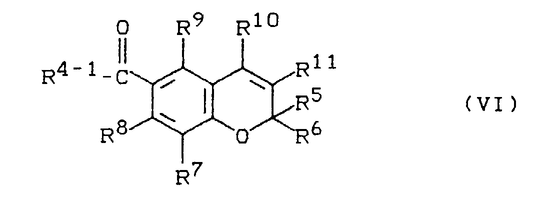 in welcher
- R⁴⁻¹: für Wasserstoff, jeweils geradkettiges oder verzweigtes Alkyl oder Alkoxy mit 1 bis 4 Kohlenstoffatomen oder Hydroxy steht und
- R⁵, R⁶, R⁷, R⁸, R⁹, R¹⁰ und R¹¹: die oben angegebene Bedeutung haben,
reduziert, vorzugsweise mittels komplexer Metallhydride wie beispielsweise Natriumborhydrid oder Lithiumaluminiumhydrid, oder mit Grignard-Verbindungen der allgemeinen Formel (VII)

R³-MgHal (VII)

in der R³ und Hal die oben angegebene Bedeutung haben, in an sich bekannter Weise umsetzt.

Die Carbonylverbindungen der Formel (VI) sind bekannt oder können in Analogie zu bekannten Verfahren hergestellt werden (vgl. Bull. Chem. Soc. Jpn. 57, 442 (1984); Indian J. Chem. 17B, 73 (1979); Angew. Chem. 94, 254 (1982).

Die zur Durchführung des erfindungsgemäßen Verfahrens (b) als Ausgangsstoffe benötigten 5-Halogenpyridazinone sind durch die Formel (IV) allgemein definiert. In dieser Formel (IV) stehen, Hal vorzugsweise für Chlor und Brom sowie R¹ und R² vorzugsweise für diejenigen Reste, die bereits im Zusammenhang mit der Beschreibung der erfindungsgemäßen Stoffe der Formel (Ia) als bevorzugt für diese Substituenten genannt wurden.

Die 5-Halogenpyridazinone der Formel (IV) sind bekannt oder erhältlich in Analogie zu bekannten Verfahren (vgl. z.B. EP 169 375; EP 302 346; Chem. Zvesti 38, 239-246 [1984] bzw. CA 101: 110848u; GB 2095 669; Synthesis 1981, 631-633.)

Die zur Durchführung des erfindungsgemäßen Verfahrens (b) weiterhin als Ausgangsstoffe benötigten Alkohole und Thiole sind durch die Formel (V) allgemein definiert. Bevorzugt sind die Ausgangsstoffe der Formel (V), in der R³, R⁴, R⁵, R⁶, R⁷, R⁸, R⁹, R¹⁰, R¹¹ und X vorzugsweise für diejenigen Reste stehen, die bereits im Zusammenhang mit der Beschreibung der erfindungsgemäßen Stoffe der Formel (Ia) als bevorzugt für diese Substituenten genannt wurden.

Als Zwischenprodukte für die Herstellung der erfindungsgemäßen Wirkstoffe der allgemeinen Formel (Ia) gemäß Herstellungsverfahren (a) und (b) werden die neuen Alkylierungsmittel der Formel (III) sowie die neuen Chromenylalkylalkohole der Formel (Va) von der Erfindung mit umfaßt.

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphonium-bromid, Trimethyl-C₁₃/C₁₅-alkylammoniumchlorid, Dibenzyl-dimethyl-ammoniummethylsulfat, Dimethyl-C₁₂/C₁₄-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxy-ethoxy)-ethyl]-amin.

Die erfindungsgemäßen Verfahren (a) und (b) werden vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und 150°C, vorzugsweise bei Temperaturen zwischen 20°C und 120°C. Zur Durchführung des erfindungsgemäßen Verfahrens (a) setzt man pro Mol an 5-Hydroxy- oder 5-Mercaptopyridazinon der Formel (II) im allgemeinen 1,0 bis 5,0 Mol, vorzugsweise 1,0 bis 3,0 Mol an Alkylierungsmittel der Formel (III) und gegebenenfalls 1,0 bis 5,0 Mol, vorzugsweise 1,0 bis 3,0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (b) setzt man pro Mol an 5-Halogenpyridazinon der Formel (IV) im allgemeinen 1,0 bis 5,0 Mol, vorzugsweise 1,0 bis 3,0 Mol an Alkohol oder Thiol der Formel (V) und gegebenenfalls 1,0 bis 5,0 Mol, vorzugsweise 1,0 bis 3,0 Mol an Reaktionshilfsmittel ein.
Die Reaktionsdurchführung, Aufarbeitung und Isolierung der Reaktionsprodukte erfolgt nach allgemein üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis,
Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Sais-setia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp,, Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten verwenden, wie beispielsweise gegen die Larven des Meerrettichblattkäfers (Phaedon cochleariae) oder gegen die Larven der Kohlschabe (Plutella xylostella) oder gegen die Larven der grünen Reiszikade (Nephotettix cincticeps) oder gegen die Larven des Heerwurms (Spodoptera frugiperda); zur Bekämpfung von pflanzenschädigenden Milben, wie beispielsweise gegen die gemeine Spinnmilbe oder die Bohnenspinnmilbe (Tetranychus urticae).

Darüber hinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Rinderzecken (Boophilus microplus) oder gegen Räudemilben (Psoroptes ovis) einsetzen.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit. Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.
Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die biologische Wirksamkeit der erfindungsgemäßen Verbindungen soll anhand der folgenden Beispiele erläutert werden.

### Herstellungsbeispiele:

### Beispiel 1

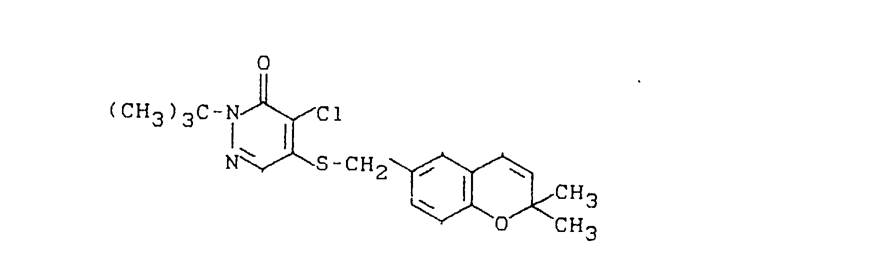

Zu einer Lösung von 2,4 g (0,011 mol) 2-tert.-Butyl-4-chlor-5-mercapto-3(2H)-pyridazinon und 2,4 g (0,012 mol) 6-Chlormethyl-2,2,-dimethyl-(2H)-chromen in 50 ml Dioxan gibt man portionsweise bei 10°C 1,4 g (0,01 mol) Kaliumcarbonat und rührt anschließend 16 Stunden bei Raumtemperatur (20°C). Anschließend wird die Reaktionsmischung mit 300 ml Wasser verdünnt und dreimal mit je 100 ml Methylenchlorid extrahiert. Die organische Phase wird über Natriumsulfat getrocknet und eingedampft. Nach Chromatographie des Rückstands über Kieselgel 60 mit Methylenchlorid erhält man 1,9 g (48% d. Th.) von 2-tert.-Butyl-4-chlor-5-[(2,2-dimethyl-(2H)-chromen-6-yl)-methylthio]-3(2H)-pyridazinon.
¹H-NMR (δppm, CDCl₃):
7,62 (1H, s), 7,1 (1H, dd), 7,0 (1H, d), 6,75 (1H, d), 6,3 (1H, d), 5,65 (1H, d), 4,17 (2H, s), 1,63 (9H, s), 1,43 (6H, s)

### Herstellung der Ausgangsverbindungen der Formel (II):

### Beispiel (II-1)

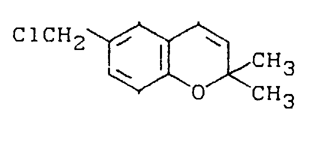

Zu einer Lösung von 5 g (0,026 mol) 6-Hydroxymethyl-2,2-dimethyl-(2H)-chromen in 100 ml Methylenchlorid tropft man 5,6 g (0,047 mol) Thionylchlorid und rührt 16 Stunden bei 40°C. Die Reaktionsmischung wird in 150 ml Wasser gegossen, die organische Phase abgetrennt, über Natriumsulfat getrocknet und eingedampft.

Man erhält 5,3 g (98% der Theorie) von 6-Chlormethyl-2,2-dimethyl-(2H)-chromen.
¹H-NMR (CDCl₃, δppm):
7,1 (dd, 1H), 7,0 (d, 1H), 6,73 (d, 1H), 6,29 (d, 1H), 5,6 (d, 1H), 4,5 (s, 2H), 1,42 (s, 6H)

### Anwendungsbeispiele

In den folgenden Anwendungsbeispielen wurden die nachstehend aufgeführten Verbindungen als Vergleichssubstanzen eingesetzt: 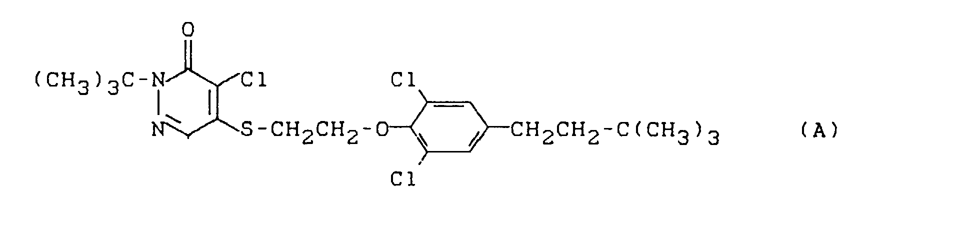 2-t-Butyl-4-chlor-5-(2-[4-(3,3-dimethyl-butyl)-2,6-dichlorphenoxy]-ethylthio)-3(2H)-pyridazinon (bekannt aus EP 232 825) 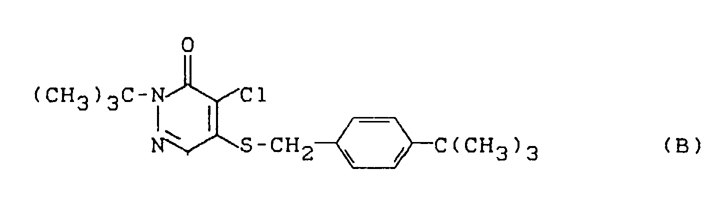 2-t-Butyl-4-chlor-5-(4-t-butyl-phenylmethylthio)-3(2H)-pyridazinon (bekannt aus EP-A 134 439) 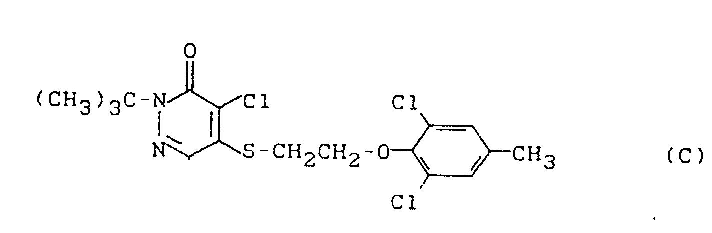 2-t-Butyl-4-chlor-5-[2-(4-methyl-2,6-dichlorphenoxy)-ethylthio]-3(2H)-pyridazinon (bekannt aus EP-A 232 825)

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigt z.B. die Verbindung des Herstellungsbeispiels (1) überlegene Wirksamkeit gegenüber dem Stand der Technik.

### Beispiel B

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung des Herstellungsbeispiels (1) überlegene Wirksamkeit gegenüber dem Stand der Technik.

### Beispiel C

### Spodoptera-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigt z.B. die Verbindung des Herstellungsbeispiels (1) überlegene Wirksamkeit gegenüber dem Stand der Technik.

### Beispiel D

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichstteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigt z.B. die Verbindung des Herstellungsbeispiels (1) überlegene Wirksamkeit gegenüber dem Stand der Technik.

### Beispiel E

### Tetranychus-Test (resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnen-spinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die Verbindung des Herstellungsbeispiels (1) überlegene Wirksamkeit gegenüber dem Stand der Technik.

### Beispiel F

### Test mit Lucilia cuprina resistent-Larven

- Emulgator:: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 Stunden wird der Abtötungsgrad bestimmt.

Bei diesem Test zeigt z.B. die Verbindung gemäß Herstellungsbeispiel (1) überlegene Wirkung gegenüber dem Stand der Technik.

### Beispiel G

### Zeckentest (Boophilus microplus)/Hemmung der Eiablage

- Lösungsmittel :: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.
In dieser Wirkstoffzubereitung werden adulte, vollgesogene Zeckenweibchen der Arten boophilus microplus (sensibel bzw. resistent) eine Minute lang getaucht. Nach dem Tauchen von je 10 weiblichen Exemplaren der verschiedenen Zeckenarten überführt man diese in Petrischalen, deren Boden mit einer entsprechend großen Filterscheibe belegt ist.

Nach 10 Tagen wird die Wirksamkeit der Wirkstoffzubereitung bestimmt durch Ermittelung der Hemmung der Eiablage gegenüber unbehandelten Kontrollzecken. Die Wirkung drückt man in Prozent aus, wobei 100 % bedeutet, daß keine Eier mehr abgelegt wurden und 0 % besagt, daß die Zecken Eier in normaler Menge ablegen.

In diesem Test zeigt z.B. die Verbindung gemäß Herstellungsbeispiel (1) überlegene Wirkung gegenüber dem Stand der Technik.

### Beispiel H (Test mit Psoroptes ovis)

- Lösungsmittel:: 35 Gewichtsteile Ethylenglykolmonomethylether
35 Gewichtsteile Nonylphenolpolyglykolether

### Testobjekt:

Natürliche Population (Larven, Nymphen, Adulte) von Psoroptes ovis, die 1 Stunde vor dem Test als Hautgeschabsel von natürlich befallenen Rindern entnommen wurden.

### Testverfahren:

Überführen von 10-25 Milben je Konzentration in die zu testende Anwendungsverdünnung, 10 und 1 ppm.
Aufbewahrung im klimatisierten Testraum (28°C ±1°C, 80% rel. Feuchte ±10%).

Wirkungskontrolle nach 24 Stunden mit dem Stereomikroskop, Vergrößerung 12,5fach.

### Kriterium:

- 3 =: 100% Wirkung = alle Milben sind abgetötet
- 2 =: >50% Wirkung = mehr als 50% der Milben sind abgetötet
- 1 =: <50% Wirkung = weniger als 50% der Milben sind abgetötet
- 0 =: 0% Wirkung = alle Milben leben

In diesem Test zeigt z.B. die Verbindung gemäß Herstellungsbeispiel (1) überlegene Wirkung gegenüber dem Stand der Technik.

## Patentansprüche

1. Substituierte Pyridazinone der Formel (Ia) 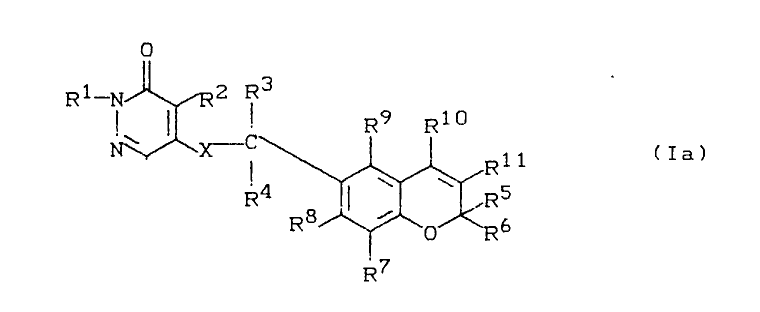 in welcher
R¹ für geradkettiges oder verzweigtes Alkyl mit 1 bis 8 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxyalkyl, (Di)alkylaminoalkyl, Alkylthioalkyl mit 1 bis 6 Kohlenstoffatomen in den jeweiligen Alkylteilen, für geradkettiges oder verzweigtes Alkenyl mit 2 bis 8 Kohlenstoffatomen, für geradkettiges oder verzweigtes 1 bis 6 Halogenatome enthaltendes Halogenalkenyl mit 2 bis 8 Kohlenstoffatomen, für jeweils gegebenenfalls einfach bis vierfach, gleich oder verschieden substituiertes Cycloalkyl mit 3 bis 7 Kohlenstoffatomen oder Cycloalkylalkyl mit 3 bis 7 Kohlenstoffatomen im Cycloalkylteil und 1 bis 4 Kohlenstoffatomen im geradkettigen oder verzweigten Alkylteil steht, wobei als Substituenten im Cycloalkylteil jeweils infrage kommen: Alkyl mit 1 bis 4 Kohlenstoffatomen und/ oder Halogen; R¹ ferner für gegebenenfalls einfach bis vierfach, gleich oder verschieden substituiertes Aryl oder Aralkyl mit 6 bis 10 Kohlenstoffatomen in den jeweiligen Arylteilen und gegebenenfalls 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
R² für Fluor, Chlor, Brom, Iod oder für geradkettiges oder verzweigtes Alkyl mit 1 bis 4 Kohlenstoffatomen steht,
R³ und R⁴ unabhängig voneinander jeweils für Wasserstoff oder für geradkettiges oder verzweigtes Alkyl mit 1 bis 4 Kohlenstoffatomen stehen,
R⁵ und R⁶ unabhängig voneinander jeweils für Wasserstoff, jeweils geradkettiges oder verzweigtes und gegebenenfalls durch Alkoxy, Carboxy, Alkoxycarbonyl, oder (Di)alkylamino substituiertes Alkyl mit jeweils 1 bis 10 Kohlenstoffatomen in den Alkylteilen, für geradkettiges oder verzweigtes Alkenyl mit 2 bis 10 Kohlenstoffatomen, für Carboxy oder für Alkoxycarbonyl mit 1 bis 4 Kohlenstoffatomen im Alkylteil, für jeweils gegebenenfalls ein-bis dreifach, gleich oder verschieden substituiertes Phenyl oder Phenylalkyl mit 1 bis 3 Kohlenstoffatomen im Alkylteil, wobei als Substituenten vorzugsweise Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio infrage kommen, stehen, oder
R⁵ und R⁶ gemeinsam mit dem Kohlenstoffatom, an das sie gebunden sind, einen gegebenenfalls substituierten drei- bis siebengliedrigen gesättigten oder ungesättigten Carbocyclus bilden,
R⁷, R⁸, R⁹, R¹⁰ und R¹¹ unabhängig voneinander jeweils für Wasserstoff, Halogen, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy, oder Halogenalkylthio mit jeweils 1 bis 4 Kohlenstoffatomen und 1 bis 9 gleichen oder verschiedenen Halogenatomen stehen und
X für Sauerstoff oder Schwefel steht.

2. Substituierte Pyridazinone der Formel (Ia) gemäß Anspruch 1, in welcher
R¹ für Methyl, Ethyl, n- oder i-Propyl, n-, i-, s-oder t-Butyl, Methoxymethyl, Ethoxymethyl, Methoxyethyl oder Ethoxyethyl, Methylthiomethyl, Ethylthiomethyl, Methylthioethyl, Ethylthioethyl, Methylaminomethyl, Methylaminoethyl, Dimethylaminomethyl, Dimethylaminoethyl, Ethylaminomethyl, Ethylaminoethyl, Diethylaminomethyl, Diethylaminoethyl, 1 bis 3 bzw. 1 bis 5 Fluor-und/oder Chloratome enthaltendes Halogenmethyl, Halogenethyl, n-oder i-Halogenpropyl, n- oder i-Halogenbutyl, Allyl, n- oder i-Butenyl, n- oder i-Pentenyl, 2-Fluorpropen-3-yl, 2-Chlorpropen-3-yl, 1-Chlorpropen-3-yl, 1,1-Dichlorpropen-3-yl, 1-Fluorpropen-3-yl, 1,1-Difluorpropen-3-yl, 1,2-Dichlorpropen-3-yl, 1,2-Difluorpropen-3-yl, 1,1,2-Trichlorpropen-3-yl oder für gegebenenfalls ein- bis vierfach gleich oder verschieden im Cycloalkylteil substituiertes Cyclopentyl, Cyclohexyl, Cyclopropylmethyl, Cyclopentylmethyl, Cyclopentylethyl, Cyclohexylmethyl oder Cyclohexylethyl steht, wobei als Substituenten jeweils infrage kommen: Fluor, Chlor, Brom, Methyl, Ethyl, Methoxy, Ethoxy, Halogenmethyl, Halogenethyl, Halogenmethoxy oder Halogenethoxy, wobei Halogen bevorzugt für Fluor und/oder Chlor steht; weiterhin für gegebenenfalls ein- oder zweifach gleich oder verschieden im Phenylteil substituiertes Phenyl, Benzyl oder Phenethyl steht, wobei als Substituenten jeweils infrage kommen: Fluor, Chlor, Brom, Cyano, Nitro, Methyl, Ethyl, n- oder i-Propyl, n-, i-, s-oder t-Butyl, Methoxy, Ethoxy, Trifuormethyl, Difluormethyl sowie Trifluormethoxy,
R² für Chlor, Brom, Methyl, Ethyl, n- oder i - Propyl steht,
R³ und R⁴ unabhängig voneinander jeweils für Wasserstoff, Methyl oder Ethyl stehen,
R⁵ und R⁶ unabhängig voneinander jeweils für Wasserstoff, Methyl, Ethyl, n- oder i-Propyl, n-, i- oder t-Butyl, n- oder i-Pentyl, n- oder i-Hexyl, Dimethoxymethyl, Carboxy, Methoxycarbonyl, Ethoxycarbonyl, n- oder i-Propoxycarbonyl, n-, i- oder t-Butoxycarbonyl, 2-Carboxy-ethyl, 2-Methoxycarbonyl-ethyl, 2-Ethoxycarbonyl-ethyl, 2-t-Butoxycarbonyl-ethyl, 4-Carboxy-butyl, 4-Methoxycarbonylbutyl, 3-Diethylamino-propyl, Allyl, 1,1-Dimethylpropen-3-yl, 1,1-Dimethylbuten-4-yl, 2-Pyrrolidinyl-ethyl, jeweils gegebenenfalls ein- bis dreifach gleich oder verschieden substituiertes Phenyl oder Phenylalkyl mit 1 bis 3 Kohlenstoffatomen im Alkylteil, wobei als Phenylsubstituenten jeweils infrage kommen: Methyl, Ethyl, n- oder i-Propyl, n-, i- oder t-Butyl, Fluor, Chlor, Brom, Methoxy, Ethoxy, Trifluormethyl, Trifluormethoxy, Difluormethoxy, Trifluormethylthio, oder
R⁵ und R⁶ gemeinsam mit dem Kohlenstoffatom, an das sie gebunden sind, einen jeweils gegebenenfalls einfach bis dreifach gleich oder verschieden substituierten Cyclopropyl-, Cyclopentyl-, Cyclohexyl-, Cyclohexenyl- oder Cycloheptylring bilden, wobei als Substituenten infraae kommen: Methyl, Ethyl, n- oder i-Propyl, n-, i- oder t-Butyl und
R⁷, R⁸, R⁹, R¹⁰ und R¹¹ unabhängig voneinander jeweils für Wasserstoff, Fluor, Chlor, Brom, Methyl, Ethyl, Methoxy, Ethoxy, Methylthio, Ethylthio, Trifluormethyl, Trifluormethoxy, Trifluormethylthio oder Difluormethoxy stehen und
X für Sauerstoff oder Schwefel steht.

3. Verfahren zur Herstellung von substituierten Pyridazinonen der allgemeinen Formel (Ia) gemäß Anspruch 1,
dadurch gekennzeichnet, daß man
entweder
(a) 5-Hydroxy- oder 5-Mercaptopyridazinone der Formel (II); 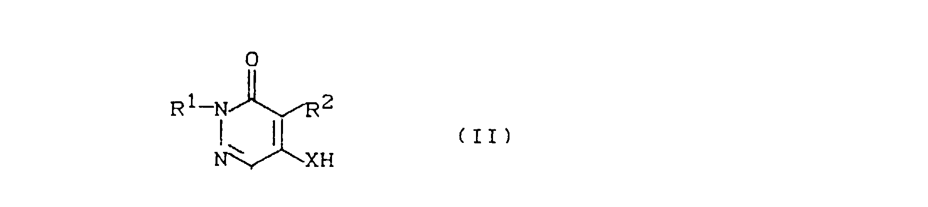 in welcher
X, R¹ und R² die in Anspruch 1 angegebene Bedeutung haben,
mit Alkylierungsmitteln der Formel (III), 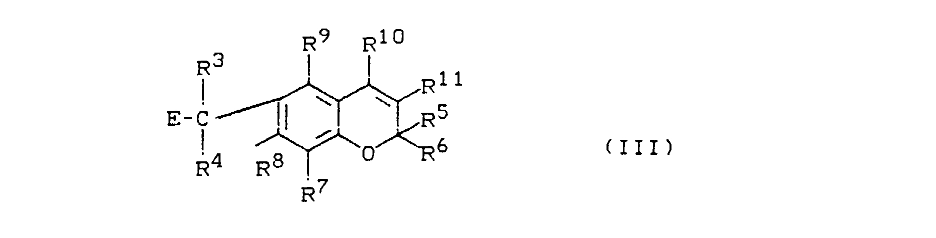 in welcher
E für eine elektronenanziehende Abgangsgruppe steht und
R³, R⁴, R⁵, R⁶, R⁷, R⁸, R⁹, R¹⁰ und R¹¹ die in Anspruch 1 angegebene Bedeutung haben,
gegebenenfalls in Gegenwart eines Verdünnungsmittels und gegebenenfalls in Gegenwart eines Reaktionshilfsmittels umsetzt oder daß man
(b) 5-Halogenpyridazinone der Formel (IV), 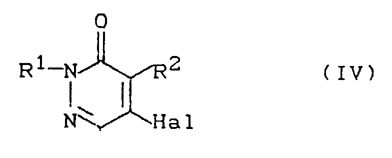 in welcher
R¹ und R² die in Anspruch 1 angegebene Bedeutung haben und
Hal für Halogen steht,
mit Alkoholen oder Thiolen der Formel (V), 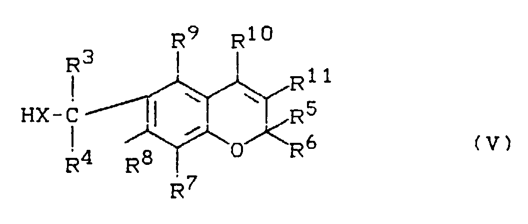 in welcher
X, R³, R⁴, R⁵, R⁶, R⁷, R⁸, R⁹, R¹⁰ und R¹¹ die in Anspruch 1 angegebene Bedeutung haben,
gegebenenfalls in Gegenwart eines Verdünnungsmittels und gegebenenfalls in Gegenwart eines Reaktionshilfsmittels umsetzt.

4. Verbindungen der Formel (III) 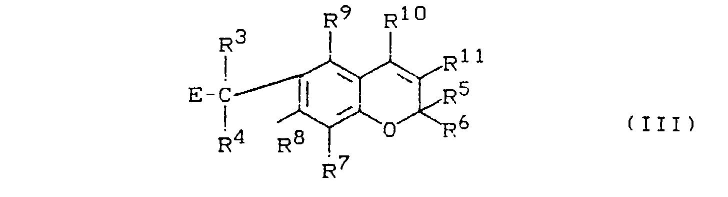 in welcher
R³, R⁴, R⁵, R⁶, R⁷, R⁸, R⁹, R¹⁰ und R¹¹ die in Anspruch 1 angegebene Bedeutung haben und
E für Halogen sowie für jeweils gegebenenfalls substituiertes Alkylsulfonyloxy, Alkoxysulfonyloxy oder Arylsulfonyloxy steht, mit der Ausnahme von 6-Chlormethyl-2H-1-benzopyran und 6-(1-Bromopropyl)-2,2-dimethyl-2H-1-benzopyran.

5. Verfahren zur Herstellung von Verbindungen der Formel (III) gemäß Anspruch 4, in welcher die Substituenten E, R³, R⁴, R⁵, R⁶, R⁷, R⁸, R⁹, R¹⁰ und R¹¹ die dort angegebene Bedeutung haben, dadurch gekennzeichnet, daß man Alkohole der Formel (Va) 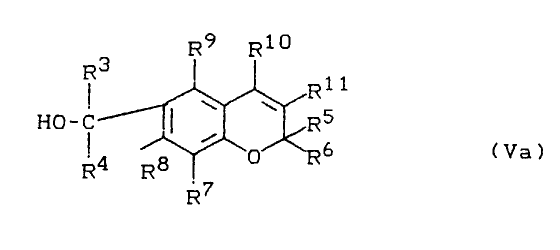 in welcher
R³, R⁴, R⁵, R⁶, R⁷, R⁸, R⁹, R¹⁰ und R¹¹ die in Anspruch 1 angegebene Bedeutung haben, mit entsprechenden Halogenierungs- bzw. Sulfonylierungsmitteln umsetzt.

6. Alkohole der Formel (Va) 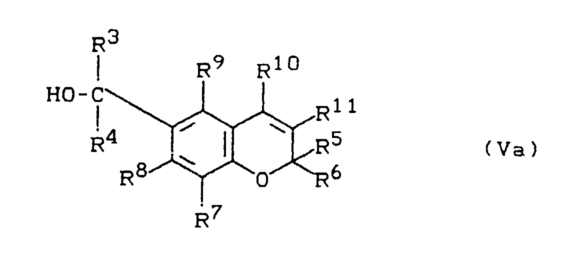 in welcher
R³, R⁴, R⁵, R⁶, R⁷, R⁸, R⁹, R¹⁰ und R¹¹ die in Anspruch 1 angegebene Bedeutung haben, mit der Maßgabe, daß R⁷, R⁸ oder R⁹ nicht für Methoxy stehen oder R⁷, R⁸, R⁹, R¹⁰ und R¹¹ nicht für Wasserstoff stehen, wenn R⁵ und R⁶ für Methyl stehen.

7. Verfahren zur Herstellung von Alkoholen der Formel (Va) gemäß Anspruch 6, in welcher R³, R⁴, R⁵, R⁶, R⁷, R⁸, R⁹, R¹⁰ und R¹¹ die dort angegebene Bedeutung haben,
dadurch gekennzeichnet, daß man Carbonylverbindungen der Formel (VI) 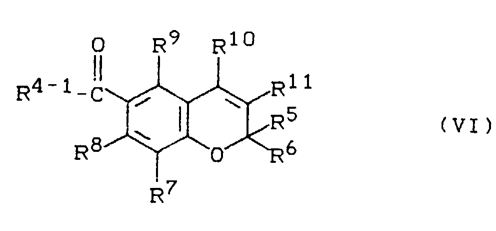 in welcher
R⁴⁻¹ für Wasserstoff, jeweils geradkettiges oder verzweigtes Alkyl oder Alkoxy mit 1 bis 4 Kohlenstoffatomen oder Hydroxy steht und
R⁵, R⁶, R⁷, R⁸, R⁹, R¹⁰ und R¹¹ die oben angegebene Bedeutung haben,
reduziert oder mit Grignard-Verbindungen der allgemeinen Formel (VII)
R³ - Mg Hal (VII)
in der R³ für Wasserstoff oder für geradkettiges oder verzweigtes Alkyl mit 1 bis 4 Kohlenstoffatomen steht
und Hal für ein Halogenatom steht,
umsetzt.

8. Schädlingsbekämpfungsmittel, gekennzeichnet durch einen Gehalt an mindestens einem substituierten Pyridazinon der Formel (Ia) gemäß Anspruch 1.

9. Verfahren zur Bekämpfung von tierischen Schädlingen, dadurch gekennzeichnet, daß man substituierte Pyridazinone der Formel (Ia) gemäß Anspruch 1 auf tierische Schädlinge und/oder deren Lebensraum einwirken läßt, ausgenommen Verfahren zur therapeutischen Behandlung des menschlichen oder tierischen Körpers.

10. Verwendung von substituierten Pyridazinonen der Formel (Ia) gemäß Anspruch 1 zur Bekämpfung von tierischen Schädlingen, ausgenommen zur therapeutischen Behandlung des menschlichen oder tierischen Körpers.

11. Verfahren zur Herstellung von Schädlingsbekämpfungsmitteln, dadurch gekennzeichnet, daß man substituierte Pyridazinone der Formel (Ia) gemäß Anspruch 1 mit Streckmitteln und/oder oberflächenaktiven Mitteln vermischt.

12. Verwendung von substituierten Pyridazinonen der Formel (Ia) gemäß Anspruch 1 zur Herstellung von Mitteln zur Bekämpfung tierischer Parasiten.

## Claims

1. Substituted pyridazinones of the formula (Ia) 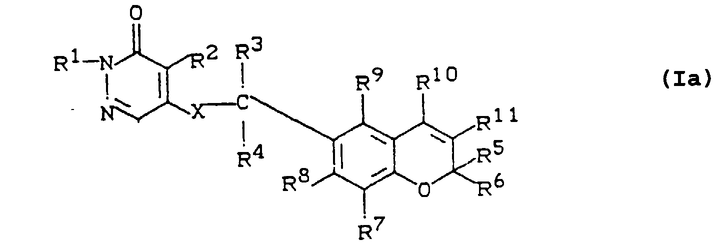 in which
R¹ represents straight-chain or branched alkyl having 1 to 8 carbon atoms, straight-chain or branched halogenoalkyl having 1 to 6 carbon atoms and 1 to 13 identical or different halogen atoms, in each case straight-chain or branched alkoxyalkyl, (di)alkylaminoalkyl and alkylthioalkyl having 1 to 6 carbon atoms in the respective alkyl moieties, straight-chain or branched alkenyl having 2 to 8 carbon atoms, straight-chain or branched halogenoalkenyl which has 2 to 8 carbon atoms and contains 1 to 6 halogen atoms, cycloalkyl which has 3 to 7 carbon atoms or cycloalkylalkyl which has 3 to 7 carbon atoms in the cycloalkyl moiety and 1 to 4 carbon atoms in the straight-chain or branched alkyl moiety, each of these cycloalkyl or cycloalkylalkyl radicals being optionally monosubstituted to tetrasubstituted by identical or different substituents, suitable substituents in the cycloalkyl moiety in each case being: alkyl having 1 to 4 carbon atoms and/or halogen; R¹ furthermore represents aryl or aralkyl which has 6 to 10 carbon atoms in the respective aryl moieties and where appropriate 1 to 4 carbon atoms in the straight-chain or branched alkyl moiety, these aryl or aralkyl radicals being optionally monosubstituted to tetrasubstituted by identical or different substituents, suitable aryl substituents being: halogen, cyano, nitro, in each case straight-chain or branched alkyl, alkoxy or alkylthio in each case having 1 to 4 carbon atoms, or in each case straight-chain or branched halogenoalkyl, halogenoalkoxy or halogenoalkylthio in each case having 1 to 4 carbon atoms and 1 to 9 identical or different halogen atoms,
R² represents fluorine, chlorine, bromine or iodine, or represents straight-chain or branched alkyl having 1 to 4 carbon atoms,
R³ and R⁴ independently of one another in each case represent hydrogen or straight-chain or branched alkyl having 1 to 4 carbon atoms,
R⁵ and R⁶ independently of one another in each case represent hydrogen, in each case straight-chain or branched alkyl which has in each case 1 to 10 carbon atoms in the alkyl moieties and which is optionally substituted by alkoxy, carboxyl, alkoxycarbonyl or (di)alkylamino, or represent straight-chain or branched alkenyl having 2 to 10 carbon atoms, carboxyl, or alkoxycarbonyl having 1 to 4 carbon atoms in the alkyl moiety, phenyl or phenylalkyl which has 1 to 3 carbon atoms in the alkyl moiety and which is in each case optionally monosubstituted to trisubstituted by identical or different substituents, preferred substituents being halogen, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio, or
R⁵ and R⁶ together with the carbon atom to which they are bonded form an optionally substituted three- to seven-membered saturated or unsaturated carbocycle,
R⁷, R⁸, R⁹, R¹⁰ and R¹¹ independently of one another in each case represent hydrogen, halogen, in each case straight-chain or branched alkyl, alkoxy or alkylthio each of which has 1 to 4 carbon atoms, or in each case straight-chain or branched halogenoalkyl, halogenoalkoxy, or halogenoalkylthio in each case having 1 to 4 carbon atoms and 1 to 9 identical or different halogen atoms, and
X is oxygen or sulphur.

2. Substituted pyridazinones of the formula (Ia) according to Claim 1, in which
R¹ represents methyl, ethyl, n- or i-propyl, n-, i-, s-or t-butyl, methoxymethyl, ethoxymethyl, methoxyethyl or ethoxyethyl, methylthiomethyl, ethylthiomethyl, methylthioethyl, ethylthioethyl, methylaminomethyl, methylaminoethyl, dimethylaminomethyl, dimethylaminoethyl, ethylaminomethyl, ethylaminoethyl, diethylaminomethyl, diethylaminoethyl, or represents halogenomethyl, halogenoethyl, n- or i-halogenopropyl, n- or i-halogenobutyl, allyl, n- or i-butenyl or n- or i-pentenyl, each of which contains 1 to 3, or 1 to 5, fluorine and/or chlorine atoms, or 2-fluoropropen-3-yl, 2chloropropen-3-yl, 1-chloropropen-3-yl, 1,1-dichloropropen-3-yl, 1-fluoropropen-3-yl, 1,1-difluoropropen-3-yl, 1,2-dichloropropen-3-yl, 1,2-difluoropropen-3-yl, 1,1, 2-trichloropropen-3-yl, or represents cyclopentyl, cyclohexyl, cyclopropylmethyl, cyclopentylmethyl, cyclopentylethyl, cyclohexylmethyl or cyclohexylethyl, each of which is optionally monosubstituted to tetrasubstituted in the cycloalkyl moiety by identical or different substituents, suitable substituents in each case being: fluorine, chlorine, bromine, methyl, ethyl, methoxy, ethoxy, halogenomethyl, halogenoethyl, halogenomethoxy or halogenoethoxy, halogen preferably representing fluorine and/or chlorine; furthermore represents phenyl, benzyl or phenethyl, each of which is optionally monosubstituted or disubstituted in the phenyl moiety by identical or different substituents, suitable substituents in each case being: fluorine, chlorine, bromine, cyano, nitro, methyl, ethyl, n- or i- propyl, n-, i-, s- or t-butyl, methoxy, ethoxy, trifluoromethyl, difluoromethyl and trifluoromethoxy,
R² represents chlorine, bromine, methyl, ethyl or n- or i-propyl,
R³ and R⁴ independently of one another in each case represent hydrogen, methyl or ethyl,
R⁵ and R⁶ independently of one another in each case represent hydrogen, methyl, ethyl, n- or i-propyl, n-, i- or t-butyl, n- or i-pentyl, n- or i-hexyl, dimethoxymethyl, carboxyl, methoxycarbonyl, ethoxycarbonyl, n- or i-propoxycarbonyl, n-, i- or t-butoxycarbonyl, 2-carboxy-ethyl, 2-methoxycarbonyl-ethyl, 2-ethoxycarbonyl-ethyl, 2-t-butoxycarbonyl-ethyl, 4-carboxy-butyl, 4-methoxycarbonyl-butyl, 3-diethylamino-propyl, allyl, 1,1-dimethylpropen-3-yl, 1,1-dimethylbuten-4-yl, 2-pyrrolidinyl-ethyl, or phenyl or phenylalkyl which has 1 to 3 carbon atoms in the alkyl moiety, each of these phenyl or phenylalkyl radicals being optionally monosubstituted to trisubstituted by identical or different substituents, suitable phenyl substituents in each case being: methyl, ethyl, n- or i-propyl, n-, i- or t-butyl, fluorine, chlorine, bromine, methoxy, ethoxy, trifluoromethyl, trifluoromethoxy, difluoromethoxy or trifluoromethylthio, or
R⁵ and R⁶ together with the carbon atom to which they are bonded form a cyclopropyl, cyclopentyl, cyclohexyl, cyclohexenyl or cycloheptyl ring, each of which is optionally monosubstituted to trisub-stituted by identical or different substituents, suitable substituents being: methyl, ethyl, n- or i-propyl or n-, i- or t-butyl, and
R⁷, R⁸, R⁹, R¹⁰ and R¹¹ independently of one another in each case represent hydrogen, fluorine, chlorine, bromine, methyl, ethyl, methoxy, ethoxy, methylthio, ethylthio, trifluoromethyl, trifluoromethoxy, trifluoromethylthio or difluoromethoxy, and
X represents oxygen or sulphur.

3. Process for the preparation of substituted pyridazinones of the general formula (Ia) according to Claim 1,
characterized in that either
(a) 5-hydroxy- or 5-mercaptopyridazinones of the formula (II), 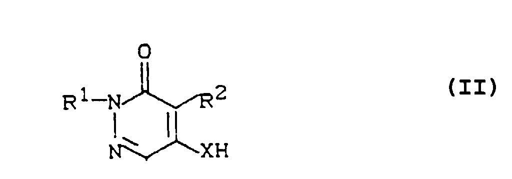 in which
X, R¹ and R² have the meaning given in Claim 1, are reacted with alkylating agents of the formula (III), 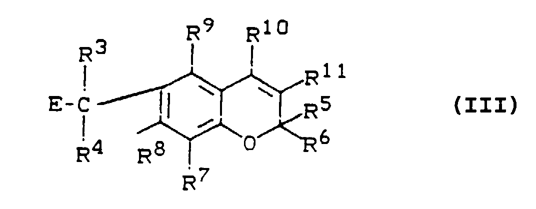 in which
E represents an electron-attracting leaving group and
R³, R⁴, R⁵, R⁶, R⁷, R⁸, R⁹, R¹⁰ and R¹¹ have the mentioned meaning given in Claim 1,
if appropriate in the presence of a diluent and if appropriate in the presence of a reaction auxiliary, or in that
(b) 5-halogenopyridazinones of the formula (IV), 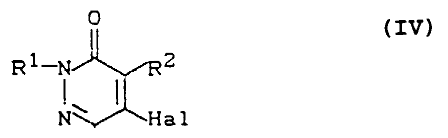 in which
R¹ and R² have the meaning given in Claim 1 and
Hal represents halogen,
are reacted with alcohols or thiols of the formula (V), 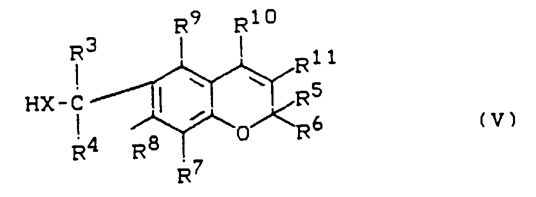 in which
X, R³, R⁴, R⁵, R⁶, R⁷, R⁸, R⁹, R¹⁰ and R¹¹ have the meaning given in Claim 1,
if appropriate in the presence of a diluent and if appropriate in the presence of a reaction auxiliary.

4. Compounds of the formula (III) 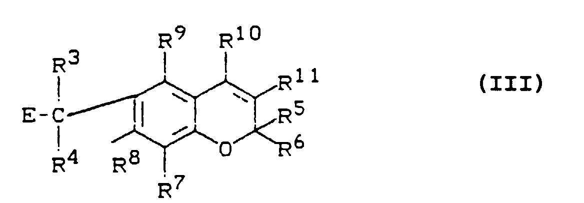 in which
R³, R⁴, R⁵, R⁶, R⁷, R⁸, R⁹, R¹⁰, and R¹¹, have the meaning given in Claim 1,
E represents halogen and also represents in each case optionally substituted alkylsulphonyloxy, alkoxysulphonyloxy or arylsulphonyloxy, with the exception of 6-chloromethyl-2H-1-benzopyran and 6-(1-bromopropyl)-2,2-dimethyl-2H-1-benzopyran.

5. Process for the preparation of compounds of the formula (III), according to Claim 4, in which the substituents E, R³, R⁴, R⁵, R⁶, R⁷, R⁸, R⁹, R¹⁰ and R¹¹ have the meaning given therein, characterized in that alcohols of the formula (Va) 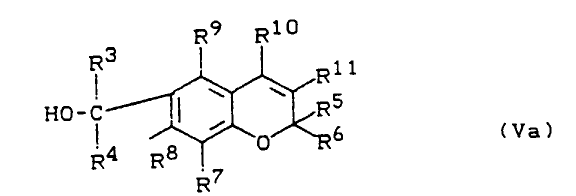 in which
R³, R⁴, R⁵, R⁶, R⁷, R⁸, R⁹, R¹⁰, and R¹¹, have the meaning given in Claim 1,
are reacted with appropriate halogenation on sulphonylation agents.

6. Alcohols of the formula (Va) 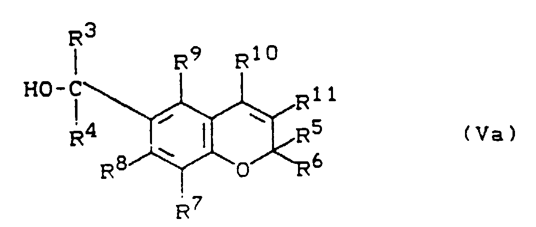 in which
R³, R⁴, R⁵, R⁶, R⁷, R⁸, R⁹, R¹⁰, and R¹¹, have the meaning given in Claim 1,
with the proviso that R⁷, R⁸ or R⁹ do not represent methoxy, or R⁷, R⁸, R⁹, R¹⁰ and R¹¹ do not represent hydrogen if R⁵ and R⁶ represent methyl.

7. Process for the preparation of alcohols of the formula (Va), according to Claim 6, in which R³, R⁴, R⁵, R⁶, R⁷, R⁸, R⁹, R¹⁰ and R¹¹ have the meaning given therein,
characterized in that carbonyl compounds of the formula (VI) 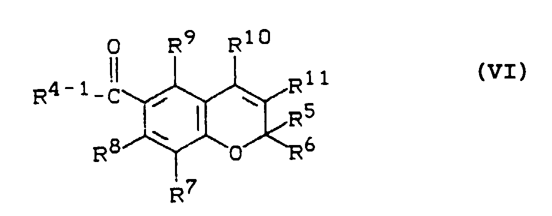 in which
R⁴⁻¹ represents hydrogen, in each case straight-chain or branched alkyl or alkoxy having 1 to 4 carbon atoms, or hydroxyl, and
R⁵, R⁶, R⁷, R⁸, R⁹, R¹⁰ and R¹¹ have the above-mentioned meaning,
are reduced or reacted with Grignard compounds of the general formula (VII)
R³ - Mg Hal (VII)
in which
R³ represents hydrogen or straight-chain or branched alkyl having 1 to 4 carbon atoms and
Hal represents a halogen atom.

8. Pesticides, characterized in that they contain at least one substituted pyridazinone of the formula (Ia) according to Claim 1.

9. Method of combating animal pests, characterized in that substituted pyridazinones of the formula (Ia) according to Claim 1 are allowed to act on animal pests and/or their habitat, with the exception of methods for therapeutic treatment of the human or animal body.

10. Use of substituted pyridazinones of the formula (Ia) according to Claim 1 for combating animal pests, except for the therapeutic treatment of the human or animal body.

11. Process for the preparation of pesticides, characterized in that substituted pyridazinones of the formula (Ia) according to Claim 1 are mixed with extenders and/or surface-active agents.

12. Use of substituted pyridazinones of the formula (Ia) according to Claim 1 for the preparation of compositions for combating animal parasites.

## Revendications

1. Pyridazinones substituées de formule (Ia) 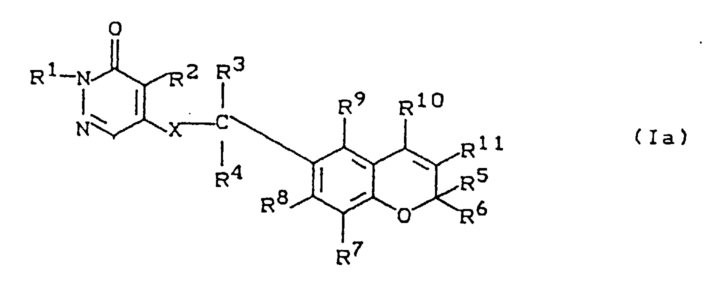 dans laquelle
R¹ représente un groupe alkyle à chaîne droite ou ramifiée contenant de 1 à 8 atomes de carbone; un groupe halogénalkyle à chaîne droite ou ramifiée contenant de 1 à 6 atomes de carbone et de 1 à 13 atomes d'halogène identiques ou différents; un groupe alcoxyalkyle, un groupe (di)alkylaminoalkyle, un groupe alkylthioalkyle contenant de 1 à 6 atomes de carbone dans les fractions alkyle respectives, chacun de ces groupes étant à chaîne droite ou ramifiée; un groupe alcényle à chaîne droite ou ramifiée contenant de 2 à 8 atomes de carbone; un groupe halogénalcényle à chaîne droite ou ramifiée contenant de 1 à 6 atomes d'halogène et de 2 à 8 atomes de carbone; un groupe cycloalkyle contenant de 3 à 7 atomes de carbone ou un groupe cycloalkylalkyle contenant de 3 à 7 atomes de carbone dans la fraction cycloalkyle et de 1 à 4 atomes de carbone dans la fraction alkyle à chaîne droite ou ramifiée, chacun de ces groupes portant éventuellement de 1 à 4 substituants identiques ou différents, dans lesquels, comme substituants dans la fraction cycloalkyle, on envisage respectivement: un groupe alkyle contenant de 1 à 4 atomes de carbone et/ou un atome d'halogène; R¹ représente en outre un groupe aryle ou un groupe aralkyle contenant de 6 à 10 atomes de carbone dans les fractions aryle respectives et éventuellement de 1 à 4 atomes de carbone dans la fraction alkyle à chaîne droite ou ramifiée, portant éventuellement de 1 à 4 substituants identiques ou différents, dans lesquels, comme substituants du groupe aryle, on envisage: un atome d'halogène, un groupe cyano, un groupe nitro; un groupe alkyle, un groupe alcoxy ou un groupe alkylthio contenant respectivement de 1 à 4 atomes de carbone, chacun de ces groupes étant à chaîne droite ou ramifiée; ou encore un groupe halogénalkyle, un groupe halogénalcoxy ou un groupe halogénalkylthio contenant respectivement de 1 à 4 atomes de carbone et de 1 à 9 atomes d'halogène identiques ou différents, chacun de ces groupes étant à chaîne droite ou ramifiée,
R² représente un atome de fluor, un atome de chlore, un atome de brome, un atome d'iode ou encore un groupe alkyle à chaîne droite ou ramifiée contenant de 1 à 4 atomes de carbone,
R³ et R⁴ représentent, indépendamment l'un de l'autre, respectivement un atome d'hydrogène ou encore un groupe alkyle à chaîne droite ou ramifiée contenant de 1 à 4 atomes de carbone,
R⁵ et R⁶ représentent, indépendamment l'un de l'autre, respectivement un atome d'hydrogène; respectivement un groupe alkyle à chaîne droite ou ramifiée contenant respectivement de 1 à 10 atomes de carbone dans les fractions alkyle et portant éventuellement un ou plusieurs substituants identiques ou différents alcoxy, carboxyle, alcoxycarbonyle ou (di)alkylamino; un groupe alcényle à chaîne droite ou ramifiée contenant de 2 à 10 atomes de carbone; un groupe carboxyle; ou encore un groupe alcoxycarbonyle contenant de 1 à 4 atomes de carbone dans la fraction alkyle; un groupe phényle ou un groupe phénylalkyle contenant de 1 à 3 atomes de carbone dans la fraction alkyle, chacun de ces groupes portant éventuellement de 1 à 3 substituants identiques ou différents, dans lesquels, comme substituants, on envisage de préférence un atome d'halogène, un groupe alkyle en C₁-C₄, un groupe halogénalkyle en C₁-C₄, un groupe alcoxy en C₁-C₄, un groupe halogénalcoxy en C₁-C₄, un groupe alkyl(en C₁-C₄) thio et un groupe halogénalkyl(en C₁-C₄) thio; ou bien
R⁵ et R⁶ forment, ensemble avec l'atome de carbone auquel ils sont liés, un radical carbocyclique triangulaire à heptagonal saturé ou insaturé portant éventuellement un ou plusieurs substituants identiques ou différents,
R⁷, R⁸, R⁹, R¹⁰ et R¹¹ représentent, indépendamment l'un de l'autre, respectivement un atome d'hydrogène, un atome d'halogène; un groupe alkyle, un groupe alcoxy ou un groupe alkylthio contenant respectivement de 1 à 4 atomes de carbone, chacun de ces groupes étant à chaîne droite ou ramifiée; ou encore un groupe halogénalkyle, un groupe halogénalcoxy ou un groupe halogénalkylthio contenant respectivement de 1 à 4 atomes de carbone et de 1 à 9 atomes d'halogène identiques ou différents, chacun de ces groupes étant à chaîne droite ou ramifiée, et
X représente un atome d'oxygène ou un atome de soufre.

2. Pyridazinones substituées de formule (Ia) selon la revendication 1, dans lesquelles
R¹ représente un groupe méthyle, un groupe éthyle, un groupe n- ou i-propyle, un groupe n-, i-, s- ou t-butyle, un groupe méthoxyméthyle, un groupe éthoxyméthyle, un groupe méthoxyéthyle ou un groupe éthoxyéthyle, un groupe méthylthiométhyle, un groupe éthylthiométhyle, un groupe méthylthioéthyle, un groupe éthylthioéthyle, un groupe méthylaminométhyle, un groupe méthylaminoéthyle, un groupe diméthylaminométhyle, un groupe diméthylaminoéthyle, un groupe éthylaminométhyle, un groupe éthylaminoéthyle, un groupe diéthylaminométhyle, un groupe diéthylaminoéthyle, un groupe halogénométhyle, un groupe halogénoéthyle, un groupe n- ou i-halogénopropyle, un groupe n- ou i-halogénobutyle contenant de 1 à 3, respectivement de 1 à 5 atomes de fluor et/ou de chlore, un groupe allyle, un groupe n- ou i-butényle, un groupe n- ou i-pentényle, un groupe 2-fluoropropén-3-yle, un groupe 2-chloropropén-3-yle, un groupe 1-chloropropén-3-yle, un groupe 1,1-dichloropropén-3-yle, un groupe 1-fluoropropén-3-yle, un groupe 1,1-difluoropropén-3-yle, un groupe 1,2-dichloropropén-3-yle, un groupe 1,2-difluoropropén-3-yle, un groupe 1,1,2-trichloropropén-3-yle; ou encore un groupe cyclopentyle, un groupe cyclohexyle, un groupe cyclopropylméthyle, un groupe cyclopentylméthyle, un groupe cyclopentyléthyle, un groupe cyclohexylméthyle ou un groupe cyclohexyléthyle portant éventuellement de 1 à 4 substituants identiques ou différents dans la fraction cycloalkyle, dans lesquels, comme substituants, on envisage respectivement: un atome de fluor, un atome de chlore, un atome de brome, un groupe méthyle, un groupe éthyle, un groupe méthoxy, un groupe éthoxy, un groupe halogénométhyle, un groupe halogénoéthyle, un groupe halogénométhoxy ou un groupe halogénoéthoxy, dans lesquels l'atome d'halogène représente de préférence un atome de fluor et/ou un atome de chlore; en outre, un groupe phényle, un groupe benzyle ou un groupe phénéthyle portant éventuellement 1 ou 2 substituants identiques ou différents dans la fraction phényle, dans lesquels, comme substituants, on envisage respectivement: un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe trifluorométhyle, un groupe difluorométhyle, ainsi qu'un groupe trifluorométhoxy,
R² représente un atome de chlore, un atome de brome, un groupe méthyle, un groupe éthyle, un groupe n- ou i-propyle,
R³ et R⁴ représentent, indépendamment l'un de l'autre, respectivement un atome d'hydrogène, un groupe méthyle ou un groupe éthyle,
R⁵ et R⁶ représentent, indépendamment l'un de l'autre, respectivement un atome d'hydrogène, un groupe méthyle, un groupe éthyle, un groupe n- ou i-propyle, un groupe n-, i- ou t-butyle, un groupe n- ou i-pentyle, un groupe n- ou i-hexyle, un groupe diméthoxyméthyle, un groupe carboxyle, un groupe méthoxycarbonyle, un groupe éthoxycarbonyle, un groupe n- ou i-propoxycarbonyle, un groupe n-, i- ou t-butoxycarbonyle, un groupe 2-carboxyéthyle, un groupe 2-méthoxycarbonyléthyle, un groupe 2-éthoxycarbonyléthyle, un groupe 2-t-butoxycarbonyléthyle, un groupe 4-carboxybutyle, un groupe 4-méthoxycarbonylbutyle, un groupe 3-diéthylaminopropyle, un groupe allyle, un groupe 1,1-diméthylpropén-3-yle, un groupe 1,1-diméthylbutén-4-yle, un groupe 2-pyrrolidinyléthyle; un groupe phényle ou un groupe phénylalkyle contenant de 1 à 3 atomes de carbone dans la fraction alkyle, chacun de ces groupes portant éventuellement de 1 à 3 substituants identiques ou différents, dans lesquels, comme substituants du groupe phényle, on envisage respectivement: un groupe méthyle, un groupe éthyle, un groupe n- ou i-propyle, un groupe n-, i- ou t-butyle, un atome de fluor, un atome de chlore, un atome de brome, un groupe méthoxy, un groupe éthoxy, un groupe trifluorométhyle, un groupe trifluorométhoxy, un groupe difluorométhoxy, un groupe trifluorométhylthio, ou bien
R⁵ et R⁶ forment, ensemble avec l'atome de carbone auquel ils sont liés, un noyau cyclopropyle, cyclopentyle, cyclohexyle, cyclohexényle ou cycloheptyle, chacun de ces noyaux portant éventuellement de 1 à 3 substituants identiques ou différents, dans lesquels, comme substituants, on envisage: un groupe méthyle, un groupe éthyle, un groupe n- ou i-propyle, un groupe n-, i- ou t-butyle, et
R⁷, R⁸, R⁹, R¹⁰ et R¹¹ représentent, indépendamment l'un de l'autre, respectivement un atome d'hydrogène, un atome de fluor, un atome de chlore, un atome de brome, un groupe méthyle, un groupe éthyle, un groupe méthoxy, un groupe éthoxy, un groupe méthylthio, un groupe éthylthio, un groupe trifluorométhyle, un groupe trifluorométhoxy, un groupe trifluorométhylthio ou un groupe difluorométhoxy, et
X représente un atome d'oxygène ou un atome de soufre.

3. Procédé pour la préparation de pyridazinones substituées répondant à la formule générale (Ia) selon la revendication 1,
caractérisé, soit en ce qu'on fait réagir
(a) des 5-hydroxy- ou 5-mercaptopyridazinones de formule (II) 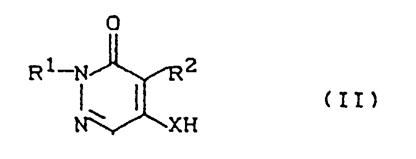 dans laquelle
X, R¹ et R² ont la signification indiquée à la revendication 1,
avec des agents d'alkylation de formule (III) 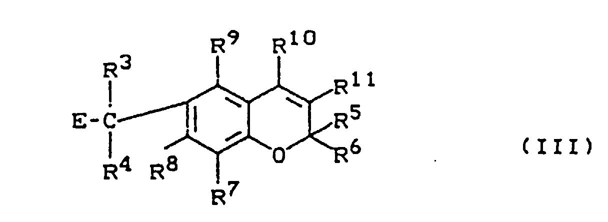 dans laquelle
E représente un groupe qui se sépare, attirant les électrons, et
R³, R⁴, R⁵, R⁶, R⁷, R⁸, R⁹, R¹⁰ et R¹¹ ont la signification indiquée à la revendication 1, éventuellement en présence d'un diluant et éventuellement en présence d'un adjuvant réactionnel, soit en ce qu'on fait réagir,
(b) des 5-halogénopyridazinones de formule (IV) 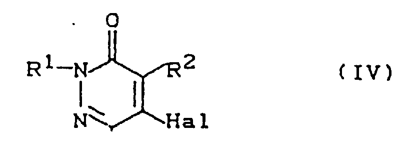 dans laquelle
R¹ et R² ont la signification indiquée à la revendication 1, et
Hal représente un atome d'halogène,
avec des alcools ou des thiols de formule (V) 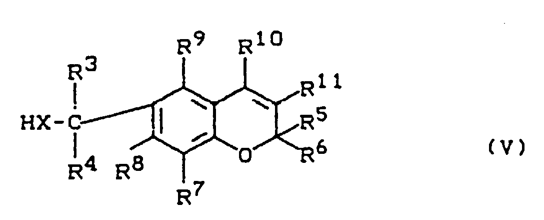 dans laquelle
x, R³, R⁴, R⁵, R⁶, R⁷, R⁸, R⁹, R¹⁰ et R¹¹ ont la signification indiquée à la revendication 1,
éventuellement en présence d'un diluant et éventuellement en présence d'un adjuvant réactionnel.

4. Composés de formule (III) 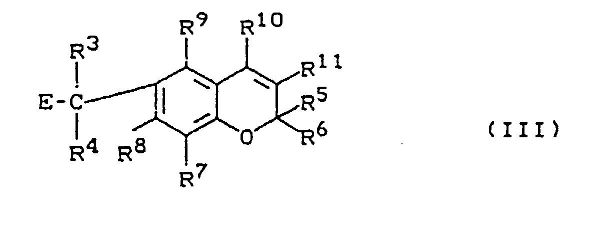 dans laquelle
R³, R⁴, R⁵, R⁶, R⁷, R⁸, R⁹, R¹⁰ et R¹¹ ont la signification indiquée à la revendication 1, et
E représente un atome d'halogène, ainsi qu'un groupe alkylsulfonyloxy, un groupe alcoxysulfonyloxy ou un groupe arylsulfonyloxy, chacun de ces groupes portant éventuellement un ou plusieurs substituants identiques ou différents, à l'exception du 6-chlorométhyl-2H-1-benzopyranne et du 6-(1-bromopropyl)-2,2-diméthyl-2H-1-benzopyranne.

5. Procédé pour la préparation de composés de formule (III) selon la revendication 4, dans lesquels les substituants E, R³, R⁴, R⁵, R⁶, R⁷, R⁸, R⁹, R¹⁰ et R¹¹ ont la signification indiquée dans cette formule, caractérisé en ce qu'on fait réagir des alcools de formule (Va) 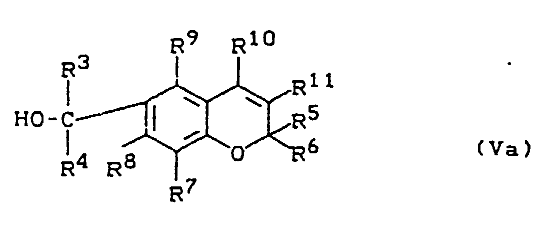 dans laquelle R³, R⁴, R⁵, R⁶, R⁷, R⁸, R⁹, R¹⁰ et R¹¹ ont la signification indiquée à la revendication 1, avec des agents d'halogénation, respectivement de sulfonylation correspondants.

6. Alcools de formule (Va) 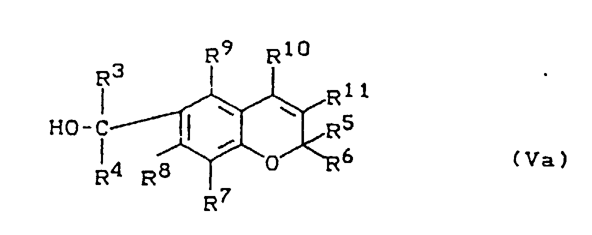 dans laquelle
R³, R⁴, R⁵, R⁶, R⁷, R⁸, R⁹, R¹⁰ et R¹¹ ont la signification indiquée à la revendication 1,
avec cette mesure que R⁷, R⁸ ou R⁹ ne représentent pas un groupe méthoxy, ou bien R⁷, R⁸, R⁹, R¹⁰ et R¹¹ ne représentent pas un atome d'hydrogène lorsque R⁵ et R⁶ représentent un groupe méthyle.

7. Procédé pour la préparation d'alcools de formule (Va) selon la revendication 6, dans lesquels R³, R⁴, R⁵, R⁶, R⁷, R⁸, R⁹, R¹⁰ et R¹¹ ont la signification indiquée dans cette formule, caractérisé en ce qu'on réduit des composés carbonylés de formule (VI) 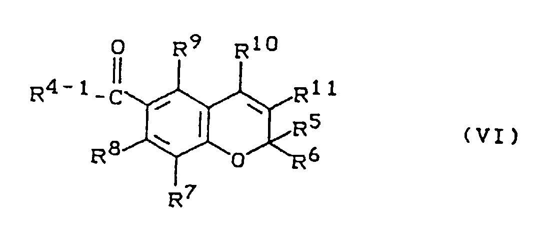 dans laquelle
R⁴⁻¹ représente un atome d'hydrogène; un groupe alkyle ou un groupe alcoxy contenant de 1 à 4 atomes de carbone, ou encore un groupe hydroxyle, chacun de ces groupes étant à chaîne droite ou ramifiée, et
R⁵, R⁶, R⁷, R⁸, R⁹, R¹⁰ et R¹¹ ont la signification indiquée ci-dessus,
ou bien on les fait réagir avec des composés de Grignard répondant à la formule générale (VII)
R³ - Mg Hal (VII)
dans laquelle
R³ représente un atome d'hydrogène ou un groupe alkyle à chaîne droite ou ramifiée contenant de 1 à 4 atomes de carbone, et Hal représente un atome d'halogène.

8. Agents de lutte contre les parasites, caractérisés par une teneur en au moins une pyridazinone substituée de formule (Ia) selon la revendication 1.

9. Procédé pour lutter contre des parasites animaux, caractérisé en ce qu'on laisse agir des pyridazinones substituées de formule (Ia) selon la revendication 1, sur des parasites animaux et/ou sur leur biotope, à l'exclusion de procédés pour le traitement thérapeutique du corps humain ou animal.

10. Utilisation de pyridazinones substituées de formule (Ia) selon la revendication 1, pour lutter contre des parasites animaux, à l'exclusion du traitement thérapeutique du corps humain ou animal.

11. Procédé pour la préparation d'agents de lutte contre les parasites, caractérisé en ce qu'on mélange des pyridazinones substituées de formule (Ia) selon la revendication 1, avec des diluants et/ou avec des agents tensioactifs.

12. Utilisation de pyridazinones substituées de formule (Ia) selon la revendication 1, pour la préparation d'agents destinés à la lutte contre des parasites animaux.
